# EUROPEAN PATENT APPLICATION

(11) **EP 4 349 806 A1**
(43) Date of publication of application: **10.04.2024**
(21) Application number: 22200419.4
(22) Date of filing: 07.10.2022
(51) Int. Cl.: C07C 43/23, C11D 3/20, A61Q 5/02, A61Q 19/00

(54) **BIO-BASED ANTIMICROBIAL COMPOUNDS**

(71) Applicant: Clariant International Ltd, 4132 Muttenz (CH)
(72) Inventor: SCHAAL, Petra, 65926 Frankfurt am Main (DE); VYBIRAL, Reinhard, 84508 Burgkirchen (DE); MUTCH, Kevin James, 65926 Frankfurt am Main (DE)
(74) Representative: Clariant Produkte (Deutschland) GmbH

(57) **Abstract**

The present invention relates to a compound of Formula (I) 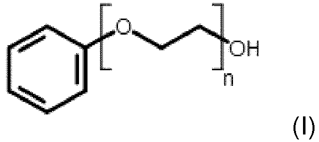 wherein n is an integer from 1 to 10; and
wherein the compound of Formula (I) has at least 10 wt-%, preferably at least 25 wt-%, more preferably at least 50 wt-%, even more preferably at least 75 wt-%, particularly preferably 100 wt-%, bio-based carbon content, relative to the total mass of carbon in the compound.

## Description

The present invention relates to bio-based antimicrobial compounds and blends, their preparation, their use as antimicrobial agents as well as formulations comprising the antimicrobial compounds and blends.

Preservation of household formulations and cosmetic formulations extends their shelf life and therefore provides greater value for money for consumers.

Furthermore, preservatives prevent consumers from distributing microbes around their home or on themselves and hence provide health benefits. Antimicrobial agents are well-described in the art and there are many available that provide excellent performance.

Many ingredients for use in cosmetic or household cleaning formulations are traditionally derived from crude oil. Environmental, economic and sustainability questions are restricting the use of products derived from this limited resource. Therefore, there is a desire to identify more sustainable and renewable, yet gentle and effective materials. Indeed, consumers are very interested in "natural" products including products with a high percentage of "natural" compounds and/or compounds that are derived from renewable materials. Consumers perceive compounds derived from natural materials to be gentler and more environmentally friendly.

Nevertheless, the availability of renewable ingredients for use in cosmetic or household cleaning formulations is still limited. There is a need for ingredients including antimicrobial agents that are not only renewable, but also provide excellent performance. There is a need for cosmetic or household cleaning ingredients including antimicrobial agents that can provide the excellent performance of modern ingredients yet are from more renewable sources.

The present invention relates to a compound of Formula (I) 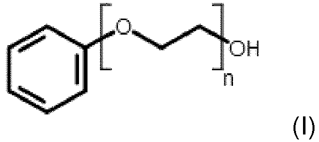
wherein n is an integer from 1 to 10; and
wherein the compound of Formula (I) has at least 10 wt-%, preferably at least 25 wt-%, more preferably at least 50 wt-%, even more preferably at least 75 wt-%, particularly preferably 100 wt-%, bio-based carbon content, relative to the total mass of carbon in the compound.

Preferably, n in Formula (I) is 1, 2 or 3. More preferably, n in Formula (I) is 1 or 2. Particularly preferably, n in Formula (I) is 1.

Particularly preferably, the compound of Formula (I) is phenoxyethanol.

In preferred embodiments, the bio-based carbon content as used herein is measured according to standard ASTM D6866-12, Method B. In preferred embodiments, the bio-based carbon content of the compound of Formula (I) as used herein is measured according to standard ASTM D6866-12, Method B. In preferred embodiments, the bio-based carbon content of ethylene oxide as used herein is measured according to standard ASTM D6866-12, Method B. In preferred embodiments, the bio-based carbon content of ethylene as used herein is measured according to standard ASTM D6866-12, Method B. In preferred embodiments, the bio-based carbon content of phenol as used herein is measured according to standard ASTM D6866-12, Method B.

The "bio-based content" is reported in ASTM D6866-12, Method B (see section 3.3.9 of ASTM D6866-12). "Bio-based carbon content", "bio-based content", "biogenic carbon content", "bio-based content", "biomass-derived carbon" herein refer to the same thing and are all measured in wt-%. Herein, the term 'bio-based carbon content' is used. ASTM D6866-12, Method B lab results report the percentage of bio-based carbon content relative to total carbon, and not to total mass of the sample or molecular weight. A comment on bio-based carbon content calculation: ASTM D6866-12, Method B (see section 9 of ASTM D6866-12) requires the percent modern carbon value (pMC) reported to be multiplied by a correction factor of 0.95 to account for excess carbon-14 in the atmosphere due to nuclear weapons testing. Hence the term "bio-based carbon content" as used herein (if measured according to standard ASTM D6866-12, Method B) is defined by the equation: Bio-based carbon content = pMC * 0.95 (%)

In preferred embodiments, the bio-based carbon content as used herein is measured according to standard ASTM D6866-21, Method B. In preferred embodiments, the bio-based carbon content of the compound of Formula (I) as used herein is measured according to standard ASTM D6866-21, Method B. In preferred embodiments, the bio-based carbon content of ethylene oxide as used herein is measured according to standard ASTM D6866-21, Method B. In preferred embodiments, the bio-based carbon content of ethylene as used herein is measured according to standard ASTM D6866-21, Method B. In preferred embodiments, the bio-based carbon content of phenol as used herein is measured according to standard ASTM D6866-21, Method B.

The "bio-based content" is reported in ASTM D6866-21, Method B. "Bio-based carbon content", "bio-based content", "biogenic carbon content", "bio-based content", "biomass-derived carbon" herein refer to the same thing and are all measured in wt-%. Herein, the term 'bio-based carbon content' is used. ASTM D6866-21, Method B lab results report the percentage of bio-based carbon content relative to total carbon, and not to total mass of the sample or molecular weight.

A review on measurement methods of bio-based carbon content for biomass-based chemicals and plastics is given by Massao Kunioka in Radioisotopes, 62, 901-925 (2013).

Bio-based products are part of the natural carbon cycle. If these products are incinerated or biodegraded, the quantity of carbon dioxide that is emitted corresponds to the quantity fixed by photosynthesis during biomass growth. Bio-based compounds of Formulae (I) can, for example, be prepared from bio-based ethylene oxide, which can in turn, for example, be prepared from bio-based ethylene. Bio-based ethylene can, for example, be obtained from bio-based ethanol by dehydration. Bio-based ethanol can, for example, be obtained from sugar by fermentation.

Bioethanol can, for example, be corn based, sugar cane based or molasses based. Bioethanol can, for example, be obtained from agricultural waste. Another ethanol source can, for example, be purple ethanol, which can be produced via CO/CO2 gas fermentation processes. Ethylene oxide can, for example, also be obtained from biomass balanced ethylene, like ethylene from recycled oils, fats or plastic waste.

In at least one embodiment, the compound of Formula (I) is derived from ethylene oxide, wherein the ethylene oxide has at least 10 wt-%, preferably at least 25 wt-%, more preferably at least 50 wt-%, even more preferably at least 75 wt-%, particularly preferably 100 wt-%, bio-based carbon content, relative to the total mass of carbon in the ethylene oxide.

Bio-based compounds of Formulae (I) can, for example, be prepared from bio-based phenol.

In at least one embodiment, the compound of Formula (I) is derived from phenol, wherein the phenol has at least 10 wt-%, preferably at least 25 wt-%, more preferably at least 50 wt-%, even more preferably at least 75 wt-%, particularly preferably 100 wt-%, bio-based carbon content, relative to the total mass of carbon in the phenol.

Bio-based compounds of Formulae (I) can, for example, be prepared from bio-based ethylene oxide and bio-based phenol.

The present invention also relates to a process for preparing a compound of Formula (I) 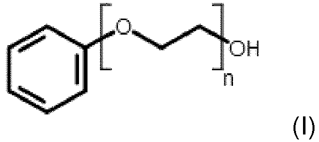
wherein n is an integer from 1 to 10; and
wherein the compound of Formula (I) has at least 10 wt-%, preferably at least 25 wt-%, more preferably at least 50 wt-%, even more preferably at least 75 wt-%, particularly preferably 100 wt-%, bio-based carbon content, relative to the total mass of carbon in the compound;
wherein the process comprises reacting phenol with ethylene oxide,
wherein
   the phenol has at least 10 wt-%, preferably at least 25 wt-%, more preferably at least 50 wt-%, even more preferably at least 75 wt-%, particularly preferably 100 wt-%, bio-based carbon content, relative to the total mass of carbon in the phenol, and/or
   the ethylene oxide has at least 10 wt-%, preferably at least 25 wt-%, more preferably at least 50 wt-%, even more preferably at least 75 wt-%, particularly preferably 100 wt-%, bio-based carbon content, relative to the total mass of carbon in the ethylene oxide.

In at least one embodiment, the phenol is reacted with the ethylene oxide in the presence of a base. Preferred bases are alkali metal or alkaline earth metal hydroxides, alkali metal or alkaline earth metal carbonates or alkali metal or alkaline earth metal hydrogen carbonates. Examples of preferred bases are sodium hydroxide, potassium hydroxide, sodium carbonate, potassium carbonate, sodium hydrogen carbonate, or potassium hydrogen carbonate.

Typically, the reaction of phenol with ethylene oxide, optionally in the presence of a base, is carried out at elevated temperatures, e.g. > 100 °C.

The present invention also relates to an antimicrobial blend comprising
(A) a compound of Formula (I) of the present invention, as component A; and
(B) an antimicrobial agent or a preservative booster, as component B.

In at least one embodiment, the weight ratio of component A to component B is from 1:20 to 20:1.

The antimicrobial blend of the present invention comprises an antimicrobial agent or a preservative booster, as component B.

In at least one embodiment, the antimicrobial blend of the present invention comprises an antimicrobial agent.

In at least one embodiment, the antimicrobial agent is selected from the group consisting of aromatic alcohols, organic acids and salts thereof, hydroxamic acids and salts thereof, compounds according to Formula (P), alkyl diols, halogenated compounds, isothiazolinones, and mixtures thereof; wherein Formula (P) is as follows: 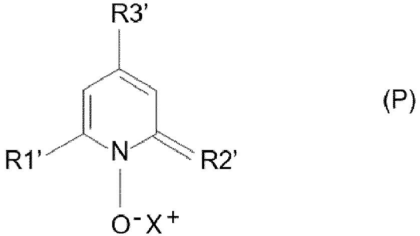 wherein
- R1': is H, an unsubstituted or halogen-substituted, branched or unbranched C₁-C₂₀-alkyl radical, an unsubstituted or halogen-substituted C₅-C₈-cycloalkyl radical, an unsubstituted or halogen-substituted C₆-C₁₀-aryl radical or an unsubstituted or halogen-substituted, branched or unbranched C₇-C₂₀-aralkyl radical;
- R2': is O or S;
- R3': is H or a C₁-C₄-alkyl radical;
- X⁺: is a cation.

Preferably, R3' is methyl.

In at least one embodiment, the antimicrobial agent is selected from the group consisting of aromatic alcohols, organic acids and salts thereof, hydroxamic acids and salts thereof, hydroxypyridones, alkyl diols, halogenated compounds, isothiazolinones, and mixtures thereof.

In at least one embodiment, the aromatic alcohols are selected from the group consisting of benzyl alcohol, veratryl alcohol, propylene phenoxyethanol, phenethyl alcohol, phenylpropanol, vanillin, 2-methyl-1-phenyl-2-propanol, hydroxyethoxyphenyl butanone, methylparaben, ethylparaben, propylparaben, and mixtures thereof.

In at least one embodiment, the organic acids and salts thereof are selected from the group consisting of benzoic acid, sorbic acid, dehydroacetic acid, lactic acid, salicylic acid, p-anisic acid, undecylenic acid, glycolic acid, propionic acid, levulinic acid, and mixtures thereof.

In at least one embodiment, the hydroxamic acid is selected from hydroxamic acids of Formula (III) 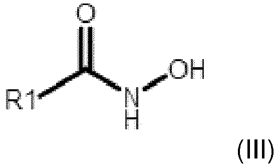 wherein R¹ is selected from saturated hydrocarbon chains having 5 to 23 carbon atoms, unsaturated hydrocarbon chains having 5 to 23 carbon atoms, and mixtures thereof.

Preferably, R¹ in Formula (III) is selected from saturated hydrocarbon chains having 5 to 17 carbon atoms, unsaturated hydrocarbon chains having 5 to 17 carbon atoms, and mixtures thereof. More preferably, R¹ in Formula (III) is selected from saturated hydrocarbon chains having 5 to 13 carbon atoms, unsaturated hydrocarbon chains having 5 to 13 carbon atoms, and mixtures thereof. Particularly preferably, R¹ in Formula (III) is selected from saturated hydrocarbon chains having 7 carbon atoms, unsaturated hydrocarbon chains having 7 carbon atoms, and mixtures thereof.

Preferred hydroxamic acids are selected from caprylhydroxamic acid, hexanohydroxamic acid, caprohydroxamic acid, laurohydroxamic acid, and mixtures thereof. A particularly preferred hydroxamic acid is caprylhydroxamic acid. Caprylhydroxamic acid may also be referred to as caprylohydroxamic acid or octanohydroxamic acid. Hydroxamic acids are described in, e.g., EP2224973.

Examples of salts of hydroxamic acids are alkali metal salts of hydroxamic acids (e.g. sodium salts of hydroxamic acids or potassium salts of hydroxamic acids) or alkaline earth metal salts of hydroxamic acids (e.g. magnesium salts of hydroxamic acids or calcium salts of hydroxamic acids).

In at least one embodiment, the compound according to Formula (P) is selected from the group consisting of 2-hydroxypyridine-N-oxide, 2-pyridinethiol-1-oxide and salts thereof, 1-hydroxy-4-methyl-6-(2,4,4-trimethylpentyl)-2(1H)-pyridone and salts thereof (preferably the monoethanolamine salt), and mixtures thereof. Formula (P) discloses and encompasses the tautomeric equivalents of these compounds, since an equilibrium always exists. In at least one embodiment, the compound according to Formula (P) is piroctone olamine (Octopirox).

In at least one embodiment, the alkyl diols are selected from the group consisting of 1,2-pentanediol, 1,2-hexanediol, 1,6-hexanediol, 1,2-octanediol, 1,2-heptanediol, 1,2-decanediol, methylpropanediol, and mixtures thereof.

In at least one embodiment, the halogenated compounds are selected from the group consisting of chlorhexidine and salts thereof, triclosan, chlorphenesin, trichlorcarban, chloroxylenol, iodoproprinyl butylcarbamate, bronopol, climbazole, and mixtures thereof.

In at least one embodiment, the isothiazolinones are selected from the group consisting of methylisothiazolinone, methylchloroisothiazolinone, benzylisothiazolinone, and mixtures thereof.

In at least one embodiment, the antimicrobial agent is selected from the group consisting of benzyl alcohol, phenethyl alcohol, benzoic acid and salts thereof, caprylhydroxamic acid, piroctone olamine, and mixtures thereof.

In a preferred embodiment, the antimicrobial agent is 1,2-octanediol, in particular bio-based 1,2-octanediol, for example bio-1,2-octanediol as disclosed in WO 2019/152569. In a preferred embodiment, the antimicrobial blend of the invention contains 90% bio-based phenoxyethanol and 10% bio-based 1,2-octanediol, i.e. bio-based phenoxyethanol and bio-based 1,2-octanediol in a ratio of 9:1.

Suitable antimicrobial agents are also listed in Annex V (updated on 28/09/2022) of the REGULATION (EC) No 1223/2009 OF THE EUROPEAN PARLIAMENT AND OF THE COUNCIL of 30 November 2009 on cosmetic products.

In at least one embodiment, the antimicrobial blend of the present invention comprises a preservative booster.

In at least one embodiment, the preservative booster is selected from the group consisting of
compounds of Formula (II) 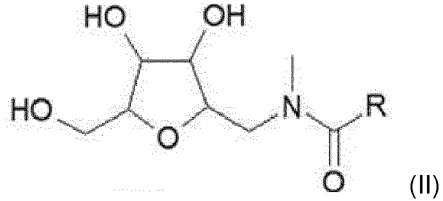
wherein R is selected from saturated hydrocarbon chains having 5 to 23 carbon atoms, unsaturated hydrocarbon chains having 5 to 23 carbon atoms, and mixtures thereof,
sorbitan esters, isosorbide esters, glyceryl ethers, and glyceryl esters.

In at least one embodiment, R in Formula (II) is selected from saturated hydrocarbon chains having 5 to 17 carbon atoms, unsaturated hydrocarbon chains having 5 to 17 carbon atoms, and mixtures thereof.

More preferably, R in Formula (II) is selected from saturated hydrocarbon chains having 5 to 13 carbon atoms, unsaturated hydrocarbon chains having 5 to 13 carbon atoms, and mixtures thereof.

Particularly preferably, R in Formula (II) is selected from saturated hydrocarbon chains having 7 to 9 carbon atoms, unsaturated hydrocarbon chains having 7 to 9 carbon atoms, and mixtures thereof.

In at least one embodiment, R in Formula (II) is selected from -(CH₂)₆CH₃, - (CH₂)₈CH₃, and mixtures thereof; or R in Formula (II) is selected from - (CH₂)₁₀CH₃, -(CH₂)₁₂CH₃, and mixtures thereof; or R in Formula (II) is selected from -(CH₂)₇CH=CH₂, -(CH₂)₇CH=CHCH₂CH₃, and mixtures thereof; or the R-C=O residue in Formula (II) is derived from coconut fatty acids; or the R-C=O residue in Formula (II) is derived from soybean fatty acids.

In at least one embodiment, R in Formula (II) is selected from -(CH₂)₆CH₃, - (CH₂)₈CH₃, and mixtures thereof. In at least one embodiment, R in Formula (II) is selected from -(CH₂)₁₀CH₃, -(CH₂)₁₂CH₃, and mixtures thereof. In at least one embodiment, R in Formula (II) is selected from -(CH₂)₇CH=CH₂, - (CH₂)₇CH=CHCH₂CH₃, and mixtures thereof. In at least one embodiment, the R-C=O residue in Formula (II) is derived from coconut fatty acids. In at least one embodiment, the R-C=O residue in Formula (II) is derived from soybean fatty acids.

As used herein, the expression "the R-C=O residue in Formula (II) is derived from coconut fatty acids" preferably means that the carbon chain length distribution in the R-C=O residue in Formula (II) corresponds to the carbon chain length distribution of the fatty acids (e.g. bound as triglycerides) in coconut oil.

As used herein, the expression "the R-C=O residue in Formula (II) is derived from soybean fatty acids" preferably means that the carbon chain length distribution in the R-C=O residue in Formula (II) corresponds to the carbon chain length distribution of the fatty acids (e.g. bound as triglycerides) in soybean oil.

Preferably, the weight ratio of compounds according to Formula (II) wherein R is - (CH₂)₆CH₃ and compounds according to Formula (II) wherein R is -(CH₂)₈CH₃ is from 1:9 to 9:1, preferably from 3:7 to 7:3.

Preferably, the weight ratio of compounds according to Formula (II) wherein R is - (CH₂)₁₀CH₃ and compounds according to Formula (II) wherein R is -(CH₂)₁₂CH₃ is from 1:9 to 9:1, preferably from 3:7 to 7:3.

Preferably, the weight ratio of compounds according to Formula (II) wherein R is - (CH₂)₇CH=CH₂ and compounds according to Formula (II) wherein R is - (CH₂)₇CH=CHCH₂CH₃ is from 1:9 to 9:1, preferably from 3:7 to 7:3.

Compounds of Formula (II) are described in, e.g., WO 2018/002100 and EP application number EP21200587.0.

In at least one embodiment, the sorbitan esters are mono-, di- or triesters of sorbitan and one or more C6-C20 fatty acids. More preferably, the sorbitan esters are mono- or diesters of sorbitan and one or more C8-C14 fatty acids. Particularly preferably, the sorbitan esters are mono- or diesters of sorbitan and caprylic acid.

Preferably, the sorbitan esters are selected from sorbitan caprylate, sorbitan stearate, sorbitan olivate, sorbitan oleate, sorbitan caprate, sorbitan laurate, sorbitan myristate, sorbitan caproate, and mixtures thereof. Particularly preferably, the sorbitan ester is sorbitan caprylate.

In at least one embodiment, the isosorbide esters are mono- or diesters of isosorbide and one or more C6-C20 fatty acids. More preferably, the isosorbide esters are mono- or diesters of isosorbide and one or more C8-C14 fatty acids. Particularly preferably, the isosorbide esters are mono- or diesters of isosorbide and caprylic acid.

Preferably, the isosorbide esters are selected from isosorbide caprylate, isosorbide stearate, isosorbide olivate, isosorbide oleate, isosorbide caprate, isosorbide laurate, isosorbide myristate, isosorbide caproate, and mixtures thereof. Particularly preferably, the isosorbide ester is isosorbide caprylate.

In at least one embodiment, the glyceryl ethers are mono- or diethers of glycerin and one or more C6-C20 fatty alcohols. More preferably, the glyceryl ethers are mono- or diethers of glycerin and one or more C8-C14 fatty alcohols. Particularly preferably, the glyceryl ethers are monoethers of glycerin and one or more C8 fatty alcohols.

Preferably, the glyceryl ethers are selected from ethylhexylglycerin, methylheptylglycerin, caprylyl glyceryl ether, and mixtures thereof.

In at least one embodiment, the glyceryl ester is glyceryl caprylate and/or glyceryl caprate, for example glyceryl caprylate/caprate.

In at least one embodiment, the blend is an aqueous solution.

In at least one embodiment, the blend comprises solvent. In at least one embodiment, the blend comprises a solvent, wherein the solvent comprises water and/or alcohol. Solvent is useful for providing the compounds used in present invention in liquid form. In at least one embodiment, the solvent is cosmetically acceptable. In at least one embodiment, the blend comprises at least 10 wt.-%, preferably at least 20 wt.-%, more preferably at least 30 wt.-%, even more preferably at least 50 wt.-% water. Water is useful for economic reasons but also because it is cosmetically acceptable. Optionally, the blend comprises water-miscible or water-soluble solvents, such as lower alkyl alcohols. In at least one embodiment, the blend comprises C1-C5 alkyl monohydric alcohols, preferably C2-C3 alkyl monohydric alcohols. The alcohols which may be present are in particular lower monohydric or polyhydric alcohols having 1 to 4 carbon atoms customarily used for cosmetic purposes, such as preferably ethanol or isopropanol.

Optionally, the blend comprises a water-soluble polyhydric alcohol. In at least one embodiment, the water-soluble polyhydric alcohols are polyhydric alcohols having two or more hydroxyl groups in the molecule. In at least one embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as pentaerythritol; pentahydric alcohols such as xylytol; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentaerythritol ether, and mixtures thereof.

In a preferred embodiment, the blend comprises a solvent selected from the group consisting of water, glycols, ethanol, and mixtures thereof. In a preferred embodiment, the blend comprises water.

In a preferred embodiment, the blend comprises an aqueous, alcoholic or aqueous-alcoholic solvent, wherein the aqueous, alcoholic or aqueous-alcoholic solvent comprises water, ethanol, propanol, isopropanol, 1,2-propylene glycol, 1,3-propylene glycol, isobutanol, butanol, butyl glycol, butyl diglycol, glycerol, or mixtures thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic solvent comprises water, isopropanol, 1,2-propylene glycol, 1,3-propylene glycol, or mixtures thereof; even more preferably wherein the aqueous, alcoholic or aqueous-alcoholic solvent consists of water or consists of a mixture of water and an alcohol wherein the alcohol is selected from the group consisting of isopropanol, 1,2-propylene glycol and 1,3-propylene glycol.

The blend of the present invention can be prepared by methods known in the art, e.g. by mixing its ingredients.

The present invention also relates to a formulation comprising
(a) from 0.01 to 10 wt-%, preferably from 0.1 to 2 wt-%, of a compound of Formula (I) of the present invention or an antimicrobial blend of the present invention; and
(b) from 90 to 99.99 wt-%, preferably from 98 to 99.9 wt-%, of one or more further components.

In at least one embodiment, the formulation is selected from the group consisting of cosmetic formulations and household cleaning formulations.

In at least one embodiment, the formulation is selected from the group consisting of shampoo, body wash, facial cleanser, face mask, bubble bath, intimate wash, bath oil, cleansing milk, micellar water, make-up remover, cleansing wipes, hair mask, perfume, liquid soap, shaving soap, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, conditioner, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, mouthwash, toothpaste, lubricating gel, moisturizer, serum, toner, aqua sorbet, cream gel, styling mousse, dry shampoo, lip stick, lip gloss, hydro-alcoholic gel, body oil, shower milk, illuminator, lip crayon, hair spray, combing cream, and sunblock.

In at least one embodiment, the formulation is a cosmetic formulation for cleansing hair and/or skin.

In at least one embodiment, the formulation is a cosmetic formulation, preferably a skin care formulation, more preferably a leave-on skin care formulation.

In at least one embodiment, the formulation is an aqueous solution.

In at least one embodiment, the formulation comprises solvent. In at least one embodiment, the formulation comprises a solvent, wherein the solvent comprises water and/or alcohol. Solvent is useful for providing the compounds used in present invention in liquid form. In at least one embodiment, the solvent is cosmetically acceptable. In at least one embodiment, the formulation comprises at least 10 wt.-%, preferably at least 20 wt.-%, more preferably at least 30 wt.-%, even more preferably at least 50 wt.-% water. Water is useful for economic reasons but also because it is cosmetically acceptable. Optionally, the formulation comprises water-miscible or water-soluble solvents, such as lower alkyl alcohols. In at least one embodiment, the formulation comprises C1-C5 alkyl monohydric alcohols, preferably C2-C3 alkyl monohydric alcohols. The alcohols which may be present are in particular lower monohydric or polyhydric alcohols having 1 to 4 carbon atoms customarily used for cosmetic purposes, such as preferably ethanol and isopropanol.

Optionally, the formulation comprises a water-soluble polyhydric alcohol. In at least one embodiment, the water-soluble polyhydric alcohols are polyhydric alcohols having two or more hydroxyl groups in the molecule. In at least one embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol (1,2-octanediol); trihydric alcohols such as glycerine, trimethylol propane, 1,2,6-hexanetriol and the like; tetrahydric alcohols such as pentaerythritol; pentahydric alcohols such as xylytol; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentaerythritol ether, and mixtures thereof.

In a preferred embodiment, the formulation comprises a solvent selected from the group consisting of water, glycols, ethanol, and mixtures thereof. In a preferred embodiment, the formulation comprises water. In a preferred embodiment, the formulation comprises 1,2-octanediol, in particular bio-based 1,2-octanediol, for example bio-1,2-octanediol as disclosed in WO 2019/152569.

In a preferred embodiment, the formulation comprises an aqueous, alcoholic or aqueous-alcoholic solvent, wherein the aqueous, alcoholic or aqueous-alcoholic solvent comprises water, ethanol, propanol, isopropanol, 1,2-propylene glycol, 1,3-propylene glycol, isobutanol, butanol, butyl glycol, butyl diglycol, glycerol, or mixtures thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic solvent comprises water, isopropanol, 1,2-propylene glycol, 1,3-propylene glycol, or mixtures thereof; even more preferably wherein the aqueous, alcoholic or aqueous-alcoholic solvent consists of water or consists of a mixture of water and an alcohol wherein the alcohol is selected from the group consisting of isopropanol, 1,2-propylene glycol and 1,3-propylene glycol.

In at least one embodiment, the formulation comprises additives common in cosmetology, pharmacy, and dermatology, which are hereinafter called auxiliaries. In at least one embodiment, the auxiliary is selected from the group consisting of oily substances, emulsifiers, coemulsifiers, cationic polymers, film formers, superfatting agents, stabilizers, active biogenic substances, glycerol, preservatives, pearlizing agents, dyes and fragrances, solvents, opacifiers, functional acids, and also protein derivatives such as gelatin, collagen hydrolysates, natural or synthetic-based polypeptides, egg yolk lecithin, lanolin and lanolin derivatives, fatty alcohols, silicones, deodorants, substances with a keratolytic and keratoplastic action, enzymes, and/or carriers/solvents.

In at least one embodiment, the formulation comprises water soluble vitamins and their derivatives, water soluble amino acids and their salts and/or derivatives, viscosity modifiers, dyes, nonvolatile solvents or diluents (water soluble and insoluble), pearlescent aids, thickeners, foam boosters, additional surfactants or nonionic co-surfactants, pediculocides, pH adjusting agents, perfumes, preservatives, chelants, proteins, skin active agents, sunscreens, UV absorbers, vitamins, niacinamide, caffeine, minoxidil, and combinations thereof. In at least one embodiment, the formulation comprises from 0 wt.-% to 5 wt.-% vitamins and amino acids, by total weight of the formulation. The formulation may also comprise pigment materials such as inorganic, nitroso, monoazo, diazo, carotenoid, triphenyl methane, triaryl methane, xanthene, quinoline, oxazine, azine, anthraquinone, indigoid, thionindigoid, quinacridone, phthalocyanine, botanical, natural colors, including: water soluble components such as those having C.I. Names. The formulation may comprise from 0 wt.-%, preferably from 0.0001 wt.-% to 5 wt.-% pigment materials.

In at least one embodiment, the formulation comprises an oily substance, which is any fatty substance which is liquid at room temperature (25°C). In at least one embodiment, the formulation comprises oily substance selected from the group consisting of silicone oils, volatile or nonvolatile, linear, branched or cyclic, optionally with organic modification; phenylsilicones; silicone resins and silicone gums; mineral oils such as paraffin oil or vaseline oil; oils of animal origin such as perhydrosqualene, lanolin; oils of plant origin such as liquid triglycerides, e.g., sunflower oil, corn oil, soybean oil, rice oil, jojoba oil, babusscu oil, pumpkin oil, grapeseed oil, sesame oil, walnut oil, apricot oil, macadamia oil, avocado oil, sweet almond oil, lady's-smock oil, castor oil, triglycerides of caprylic/capric acids, olive oil, peanut oil, rapeseed oil, argan oil, abyssinian oil, and coconut oil; synthetic oils such as purcellin oil, isoparaffins, linear and/or branched fatty alcohols and fatty acid esters, preferably guerbet alcohols having 6 to 18, preferably 8 to 10, carbon atoms; esters of linear (C₆-C₁₃) fatty acids with linear (C₆-C₂₀) fatty alcohols; esters of branched (C₆-C₁₃) carboxylic acids with linear (C₆-C₂₀) fatty alcohols, esters of linear (C₆-C₁₈) fatty acids with branched alcohols, especially 2-ethylhexanol; esters of linear and/or branched fatty acids with polyhydric alcohols (such as dimerdiol or trimerdiol, for example) and/or guerbet alcohols; triglycerides based on (C₆-C₁₀) fatty acids; esters such as dioctyl adipate, diisopropyl dimer dilinoleate; propylene glycols/dicaprylate or waxes such as beeswax, paraffin wax or microwaxes, alone or in combination with hydrophilic waxes, such as cetylstearyl alcohol, for example; fluorinated and perfluorinated oils; fluorinated silicone oils; mixtures of the aforementioned compounds.

In at least one embodiment, the formulation comprises a non-ionic coemulsifier.

In at least one embodiment, the non-ionic coemulsifier is selected from adducts of from 0 to 30 mol of ethylene oxide and/or from 0 to 5 mol of propylene oxide with linear fatty alcohols having 8 to 22 carbon atoms, with fatty acids having 12 to 22 carbon atoms, with alkylphenols having 8 to 15 carbon atoms in the alkyl group, and with sorbitan or sorbitol esters; (C₁₂-C₁₈) fatty acid monoesters and diesters of adducts of from 0 to 30 mol of ethylene oxide with glycerol; glycerol monoesters and diesters and sorbitan monoesters and diesters of saturated and unsaturated fatty acids having 6 to 22 carbon atoms and, where appropriate, their ethylene oxide adducts; adducts of from 15 to 60 mol of ethylene oxide with castor oil and/or hydrogenated castor oil; polyol esters and especially polyglycerol esters, such as polyglyceryl polyricinoleate and polyglyceryl poly-12-hydroxystearate, for example. Likewise suitable are mixtures of compounds from one or more of these classes of substance. Examples of suitable ionogenic coemulsifiers include anionic emulsifiers, such as mono-, di- or tri-phosphoric esters, but also cationic emulsifiers such as mono-, di-, or tri-alkyl quats and their polymeric derivatives.

In at least one embodiment, the formulation comprises a cationic polymer. Suitable cationic polymers include those known under the INCI designation "Polyquaternium", especially Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, and also Polyquaternium 37 & mineral oil & PPG trideceth (Salcare SC95), PVP-dimethylaminoethyl methacrylate copolymer, guar-hydroxypropyltriammonium chlorides, and also calcium alginate and ammonium alginate. It is additionally possible to employ cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as amidomethicones, for example; copolymers of adipic acid and
dimethylaminohydroxypropyldiethylenetriamine; polyaminopolyamide and cationic chitin derivatives, such as chitosan, for example.

In at least one embodiment, the formulation comprises a superfatting agent.

As superfatting agents it is possible to use substances such as, for example, polyethoxylated lanolin derivatives, lecithin derivatives, polyol fatty acid esters, monoglycerides, or fatty acid alkanol amides, the latter serving simultaneously as foam stabilizers. Moisturizers available include for example isopropyl palmitate, glycerol and/or sorbitol.

In at least one embodiment, the formulation comprises a stabilizer. As stabilizer it is possible to use metal salts of fatty acids, such as magnesium, aluminum and/or zinc stearate, for example.

In at least one embodiment, the formulation comprises a care additive. The formulations can be blended with conventional ceramides, pseudoceramides, fatty acid N-alkylpolyhydroxyalkyl amides, cholesterol, cholesterol fatty acid esters, fatty acids, triglycerides, cerebrosides, phospholipids, panthenol and similar substances as a care additive.

In at least one embodiment, the formulation comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, zinc pyrithione, piroctone, piroctone olamine (octopirox), and combinations thereof. In at least one embodiment, the formulation comprises a total amount of anti-fungal substance in the formulation of from 0.1 wt.-% to 1 wt.-%. In at least one embodiment, the formulation comprises pyridinethione anti-dandruff particulates. For example, 1-hydroxy-2-pyridinethione salts are highly preferred particulate anti-dandruff agents. The concentration of pyridinethione antidandruff particulate may range from 0.1 wt.-% to 4 wt.-%, by total weight of the formulation, preferably from 0.1 wt.-% to 3 wt.-%, more preferably from 0.3 wt.-% to 2 wt.-%. Preferred pyridinethione salts include those formed from heavy metals such as zinc, tin, cadmium, magnesium, aluminum or zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable.

Functional acids are acidic substances used to impart a clinical functionality to the skin or hair upon application. Suitable functional acids include alpha-hydroxy acids, beta-hydroxy acids, lactic acid, retinoic acid, and similar substances.

In at least one embodiment, the formulation comprises an astringent. In at least one embodiment, the astringent is selected from the group consisting of magnesium oxide, aluminum oxide, titanium dioxide, zirconium dioxide, zinc oxide, oxide hydrates, aluminum oxide hydrate (boehmite) and hydroxide, chlorohydrates of calcium, magnesium, aluminum, titanium, zirconium or zinc. In at least one embodiment, the formulation comprises from 0.001 wt.-% to 10 wt.-%, or from 0.01 wt.-% to 9 wt.-%, or from 0.05 wt.-% to 8 wt.-%, or from 0.1 wt.-% to 5 wt.-% astringent.

In at least one embodiment, the formulation comprises a deodorizing agent. In at least one embodiment, the deodorizing agent is selected from the group consisting of allantoin, bisabolol, and combinations thereof. In at least one embodiment, the formulation comprises from 0.001 wt.-% to 10 wt.-%, or from 0.01 wt.-% to 9 wt.-%, or from 0.05 wt.-% to 8 wt.-%, or from 0.1 wt.-% to 5 wt.-% deodorizing agent.

In at least one embodiment, the formulation comprises a sun protection agent and/or UV filter. Suitable sun protection agents and UV filters are disclosed in WO-2013/017262A1, from page 32, line 11 to the end of page 33. In at least one embodiment, the sun protection agent and/or UV filter is selected from the group consisting of 4-amino benzoic acid, 3-(4'-trimethylammonium)-benzylide-boran-2-one-methylsulfate, camphor benzalkonium methosulfate, 3,3,5-trimethyl-cyclohexylsalicylate, 2-hydroxy-4-methoxybenzophenone, 2-phenylbenzimidazole-5-sulfonic acid and potassium-, sodium- und triethanolamine salts thereof, 3,3'-(1,4-phenylene dimethine)-bis-(7,7-dimethyl-2-oxobicyclo[2.2.1]-heptane-1-methane sulfonic acid) and its salts, 1-(4-tert.-butylphenyl)-3-(4-methoxyphenyl)propan-1,3-dion, 3-(4'-sulfo)-benzylidene-bornane-2-one its salts, 2-cyan-3,3-diphenylacrylic acid-(2-ethylhexylester), polymers of N-[2(and 4)-(2-oxoborn-3-ylidenmethyl)benzyl]-acrylamide, 4-methoxy-cinnamic acid-2-ethyl-hexylester, ethoxylated ethyl-4-amino-benzoate, 4-methoxy-cinnamic acid-isoamylester, 2,4,6-tris-[p-(2-ethylhexyloxycarbonyl)anilino]-1,3,5-triazine, 2-(2H-benzotriazole-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)diimino]bis-(benzoic acid-2-ethylhexylester), 3-benzophenone, 4-benzophenone, 3(4'-methylbenzyliden)-D,L-camphor, 3-benzylidene-camphor, salicylic acid-2-ethylhexylester, 4-dimethyl aminobenzic acid-2-ethylhexylester, hydroxy-4-methoxybenzophenone-5 sulfonic acid and the sodium salt thereof, 4-isopropyl benzylsalicylate, N,N,N-trimethyl-4-(2-oxoborn-3-ylidenemethyl) anilium methyl sulfate, homosalate (INN), oxybenzone (INN), 2-phenylbenzimidazole-5-sulfonic acid and its sodium, potassium, and triethanolamine salts, octylmethoxy cinnamic acid, isopentyl-4-methoxy cinnamic acid, isoamyl-p-methoxy cinnamic acid, 2,4,6-trianilino-(p-carbo-2'-ethylhexyl-1'-oxy)-1,3,5-triazine (octyl triazone) phenol, 2,2(2H-benzotriazol-2-yl)-4-methyl-6-(2-methyl-3-(1,3,3,3-tetramethyl-1-(trimethylsilyl)oxy)-disiloxanyl)propyl (drometrizole trisiloxane) benzic acid, 4,4-((6-(((1,1-dimethylethyl)amino)carbonyl)phenyl)amino)-1,3,5-triazine-2,4-diyl)diimino)bis,bis(2-ethylhexyl)ester) benzoic acid, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-methylbenzylidene)-D,L-camphor (4-methylbenzylidene camphor), benzylidene-camphor-sulfonic acid, octocrylene, polyacrylamidomethyl-benzylidene-camphor, 2-ethylhexyl salicylate (octyl salicylate), 4-dimethyl-aminobenzoeacidethyl-2-hexylester (octyl dimethyl PABA), PEG-25 PABA, 2 hydroxy-4-methoxybenzo-phenone-5-sulfonic acid (5-benzophenone) and the sodium salt thereof, 2,2'-methylene-bis-6-(2H-benzotriazol-2-yl)-4-(tetramethyl-butyl)-1,1,3,3-phenol, the sodium salt of 2-2'-bis-(1,4-phenylene)1H-benzimidazole-4,6-disulfonic acid, (1,3,5)-triazine-2,4-bis((4-(2-ethyl-hexyloxy)-2-hydroxy)-phenyl)-6-(4-methoxyphenyl), 2-ethylhexyl-2-cyano-3,3-diphenyl-2-propenoate, glyceryl octanoate, di-p-methoxy cinnamic acid, p-aminobenzoic acid and its ester, 4-tert-butyl-4'-methoxydibenzoylmethane, 4-(2-β-glucopyranoxy)propoxy-2-hydroxybenzophenone, octyl salicylate, methyl-2,5-diisopropyl cinnamic acid, cinoxate, dihydroxy-dimethoxybenzophenone, disodium salts of 2,2'-dihydroxy-4,4'-dimethoxy-5,5'-disulfobenzophenone, dihydroxybenzophenone, 1,3,4-dimethoxyphenyl-4,4-dimethyl-1,3-pentanedione, 2-ethylhexyl-dimethoxybenzyliden-dioxoimidazolidinpropionate, methylene-bis-benztriazolyl tetramethylbutylphenol, phenyldibenzimidazoltetrasulfonate, bis-ethylhexyloxyphenol-methoxyphenol-triazine, tetrahydroxybenzophenone, terephthalylidendicamphor-sulfonic acid, 2,4,6-tris[4,2-ethylhexyloxycarbonyl)anilino]-1,3,5-triazine, methyl-bis(trimethylsiloxy)silyl-isopentyl trimethoxy cinnamic acid, amyl-p-dimethylaminobenzoate, amyl-p-dimethylamino benzoate, 2-ethylhexyl-p-dimethylaminobenzoate, isopropyl-p-methoxy cinnamic acid/diisopropyl cinnamic acid ester, 2-ethylhexyl-p-methoxy cinnamic acid, 2-hydroxy-4-methoxy benzophenone, 2-hydroxy-4-methoxybenzophenone-5-sulfonic acid and the trihydrate, 2-hydroxy-4-methoxybenzophenone-5-sulfonate sodium salt, phenyl-benzimidazole sulfonic acid, and combinations thereof. In at least one embodiment, the formulation comprises from 0.001 wt.-% to 10 wt.-%, preferably from 0.05 wt.-% to 5 wt.-%, even more preferably from 0.1 wt.-% to 3 wt.-%, most preferably from 0.05 wt.-% to 1 wt.-% sun protection agent and/or UV filter. In at least one embodiment, the formulation comprises a photoprotective substance in an amount of from 0.01 to 10 wt.-%, or from 0.1 to 5 wt.-%, more preferably from 0.2 to 2 wt.-%. Suitable photoprotective substances include, in particular, all of the photoprotective substances specified in EP1084696A1, which is incorporated herein by reference. In a preferred embodiment, the photoprotective substance is selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, and combinations thereof.

In at least one embodiment, the formulation comprises an anti-oxidant. In at least one embodiment, the anti-oxidant is selected from the group consisting of amino acids, peptides, sugars, imidazoles, carotinoids, carotenes, chlorogenic acid, lipoic acid, thiols, thiol glycosyl esters, thiol N-acetyl esters, thiol methyl esters, thiol ethyl esters, thiol propyl esters, thiol amyl esters, thiol butyl esters, thiol lauryl esters, thiol palmitoyl esters, thiol oleyl esters, thiol linoleyl esters, thiol cholesteryl esters, thiol glyceryl esters, dilaurylthiodipropionate, distearylthiodipropionate, thiodipropionic acid, metal chelators, hydroxy acids, fatty acids, folic acids, vitamin C, tocopherol, vitamin A, stilbenes, derivatives and combinations thereof. In at least one embodiment, the anti-oxidant is selected from the group consisting of glycine, histidine, tyrosine, tryptophan, urocaninic acid, D,L-carnosine, D-carnosine, L-carnosine, beta-carotene, alpha-carotene, lycopene, dihydrolipoic acid, aurothioglucose, propylthiouracil, thioredoxine, glutathione, cysteine, cystine, cystamine, buthioninsulfoximine, homocysteinsulfoximine, buthioninsulfone, penta-, hexa-, heptathioninsulfoximine, hydroxyfatty acids, palmitic acid, phytinic acid, lactoferrin, citric acid, lactic acid, malic acid, humic acid, bile acid, bilirubin, biliverdin, EDTA, EGTA, linoleic acid, linolenic acid, oleic acid, butylhydroxyanisol, trihydroxybutyrophenone, ubichinon, ubichinol, ascorbylpalmitate, Mg-ascorbylphosphate, ascorbylacetate, vitamin E acetate, vitamin A palmitate, carnosine, mannose, ZnO, ZnSO4, selenium methionine, stilbenes, superoxide dismutase, and combinations thereof. In at least one embodiment, the antioxidant is selected from the group consisting of vitamin A, vitamin A derivatives, vitamin E, vitamin E derivatives, and combinations thereof. In at least one embodiment, the formulation comprises from 0.001 wt.-% to 10 wt.-%, preferably from 0.05 wt.-% to 5 wt.-%, particularly preferably from 0.1 wt.-% to 3 wt.-%, also particularly preferably from 0.05 wt.-% to 1 wt.-% antioxidant.

In at least one embodiment, the formulation comprises a dye or pigment. In at least one embodiment, the formulation comprises at least one pigment. Suitable dyes and pigments are disclosed in WO2013/017262A1 in the table spanning pages 36 to 43. These may be colored pigments which impart color effects to the product mass or to hair, or they may be luster effect pigments which impart luster effects to the product mass or to hair. The color or luster effects on hair are preferably temporary, i.e. they last until the next hair wash and can be removed again by washing the hair with customary shampoos. In at least one embodiment, the formulation comprises a total amount of from 0.01 wt.-% to 25 wt.-%, preferably from 5 wt.-% to 15 wt.-% pigment. In at least one embodiment, the particle size of the pigment is from 1 micron to 200 micron, preferably from 3 micron to 150 micron, more preferably from 10 micron to 100 micron. The pigments are colorants which are virtually insoluble in the application medium, and may be inorganic or organic. Inorganic-organic mixed pigments are also possible. Preference is given to inorganic pigments. The advantage of inorganic pigments is their excellent resistance to light, weather and temperature. The inorganic pigments may be of natural origin. In at least one embodiment, the inorganic pigment is selected from the group consisting of chalk, ochre, umber, green earth, burnt sienna, graphite, and combinations thereof. The pigments may be white pigments, such as, for example, titanium dioxide or zinc oxide, black pigments, such as, for example, iron oxide black, colored pigments, such as, for example, ultramarine or iron oxide red, luster pigments, metal effect pigments, pearlescent pigments, and fluorescent or phosphorescent pigments, where preferably at least one pigment is a colored, nonwhite pigment. In at least one embodiment, the pigment is selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments), and combinations thereof. In at least one embodiment, the pigment is selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminum sulfosilicates, CI 77007, Pigment Blue 29), chromium oxide hydrate (CI 77289), Prussian blue (ferric ferrocyanide, CI 77510), carmine (cochineal), and combinations thereof. In at least one embodiment, the pigment is selected from the group consisting of pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine etc. and where the color can be determined by varying the layer thickness. Such pigments are sold, for example, under the trade names Rona^{®}, Colorona^{®}, Dichrona^{®} and Timiron^{®} by Merck. In at least one embodiment, the pigment is selected from the group consisting of organic pigments such as sepia, gamboge, bone charcoal, Cassel brown, indigo, chlorophyll and other plant pigments. In at least one embodiment, the pigment is selected from the group consisting of synthetic organic pigments such as azo pigments, anthraquinoids, indigoids, dioxazine, quinacridone, phthalocyanine, isoindolinone, perylene and perinone, metal complex, alkali blue and diketopyrrolopyrrole pigments.

In at least one embodiment, the formulation comprises from 0.01 wt.-% to 10 wt.-%, preferably from 0.05 wt.-% to 5 wt.-%, of at least one particulate substance. Suitable substances are, for example, substances which are solid at room temperature (25°C) and are in the form of particles. In at least one embodiment, the particulate substance is selected from the group consisting of silica, silicates, aluminates, clay earths, mica, insoluble salts, in particular insoluble inorganic metal salts, metal oxides, e.g. titanium dioxide, minerals and insoluble polymer particles are suitable. The particles may be present in the formulation in undissolved, preferably stably dispersed form, and, following application to the keratin substrate and evaporation of the solvent, can deposit on the substrate in solid form. A stable dispersion can be achieved by providing the formulation with a yield point which is large enough to prevent the solid particles from sinking. An adequate yield point can be established using suitable gel formers in a suitable amount. In at least one embodiment, the particulate substance is selected from the group consisting of silica (silica gel, silicon dioxide) and metal salts, in particular inorganic metal salts, where silica is particularly preferred. Metal salts are, for example, alkali metal or alkaline earth metal halides, such as sodium chloride or potassium chloride; alkali metal or alkaline earth metal sulfates, such as sodium sulfate or magnesium sulfate.

In at least one embodiment, the formulation comprises a direct dye. Preferred among the direct dyes are the following compounds, alone or in combination with one another: Hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxyethyl)amino-2-nitro-4-[di(2-hydroxyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. Particularly preferred among the aforesaid direct dyes are the following compounds, alone or in combination with one another: hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydro-xyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)-amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. In at least one embodiment, the total quantity of direct dyes in the formulation amounts to 0.01 to 15 wt.-%, preferably 0.1 to 10 wt.-%, most preferred 0.5 to 8 wt.-%.

In at least one embodiment, the formulation comprises a conditioning agent. In at least one embodiment, the conditioning agent is a water insoluble, water dispersible, non-volatile, liquid that forms emulsified, liquid particles. In at least one embodiment, the conditioning agent is a silicone (e.g., silicone oil, cationic silicone, silicone gum, high refractive silicone, or silicone resin), an organic conditioning oil (e.g., hydrocarbon oils, polyolefins, or fatty esters), a cationic conditioning surfactant, a high melting point fatty compound, or combinations thereof.

In at least one embodiment, the conditioning agent is a silicone, and the formulation comprises from 0.01 % to 10 %, or from 0.1 % to 5 % silicone conditioning agent, by total weight of the formulation. Suitable silicone conditioning agents, and optional suspending agents for the silicone, are described in US-5,104,646. In at least one embodiment, the formulation comprises a silicone gum selected from the group consisting of polydimethylsiloxane, (polydimethylsiloxane) (methylvinylsiloxane) copolymer, poly(dimethylsiloxane) (diphenylsiloxane) (methylvinylsiloxane) copolymer, and mixtures thereof.

In at least one embodiment, the formulation comprises a terminal aminosilicone. "Terminal aminosilicone" as defined herein means silicone comprising one or more amino groups at one or both ends of the silicone backbone. In at least one embodiment, the formulation is substantially free of any silicone compound comprising pendant amino groups. In an embodiment, the formulation is substantially free of any silicone compound other than terminal aminosilicones. In at least one embodiment, the amino group at at least one terminus of the silicone backbone of the terminal aminosilicone is selected from the group consisting of primary amines, secondary amines and tertiary amines. In at least one embodiment, the formulation comprises a terminal aminosilicone conforming to Formula (S):

(R^{F})ₐG₃₋ₐ-Si-(-OSiG₂)ₙ-O-SiG₃-ₐ(R^{F})ₐ (S)

wherein
- G: is hydrogen, phenyl, hydroxy, or C₁-C₈ alkyl, preferably methyl;
- a: is an integer having a value from 1 to 3, preferably 1;
- b: is 0, 1 or 2, preferably 1;
- n: is a number from 0 to 1,999;
- R^{F}: is a monovalent radical conforming to the general formula C_{q}H_{2q}L, wherein q is an integer having a value from 2 to 8 and L is selected from the following groups: -N(R^{T})CH₂-CH₂-N(R^{T})₂; -N(R^{T})₂; -N(R^{T})₃A⁻;
-N(R^{T})CH₂-CH₂-NR^{T}H₂A⁻; wherein R^{T} is hydrogen, phenyl, benzyl, or a saturated hydrocarbon radical, preferably an alkyl radical having from 1 to 20 carbon atoms; A⁻ is a halide ion.

In at least one embodiment, the terminal aminosilicone corresponding to Formula (S) has a = 1, q = 3, G = methyl, n is from 1000 to 2500, alternatively from 1500 to 1700, and L is -N(CH₃)₂. A suitable terminal aminosilicone corresponding to Formula (S) has a = O, G = methyl, n is from 100 to 1500, or from 200 to 800, and L is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is hydrogen, phenyl, benzyl, or a saturated hydrocarbon radical, preferably an alkyl radical from having from 1 to 20 carbon atoms; A⁻ is a halide ion, alternatively L is -NH₂. In at least one embodiment, the terminal aminosilicone is selected from the group consisting of bis-aminomethyl dimethicone, bis-aminoethyl dimethicone, bis-aminopropyl dimethicone, bis-aminobutyl dimethicone, and mixtures thereof. In an embodiment, the viscosity of the terminal aminosilicone is from 1,000 to 30,000 cPs, or from 5,000 to 20,000 cPs, measured at 25°C. In at least one embodiment, the formulation comprises from 0.1 % to 20 %, or from 0.5 % to 10 %, or from 1 % to 6 % terminal aminosilicone, by total weight of the formulation.

In at least one embodiment, the formulation comprises a high melting point fatty compound. The high melting point fatty compound has a melting point of 25°C or higher. In at least one embodiment, the high melting point fatty compound is selected from the group consisting of a fatty alcohol, fatty acid, fatty alcohol derivative, fatty acid derivative, and mixtures thereof. Non-limiting examples of the high melting point compounds are found in International Cosmetic Ingredient Dictionary, Fifth Edition, 1993, and CTFA Cosmetic Ingredient Handbook, Second Edition, 1992. The formulation may comprise from 0.1 % to 40 %, or from 1 % to 30 %, or from 1.5 % to 16 %, or from 1.5 % to 8 % of a high melting point fatty compound, by total weight of the formulation. This is advantageous in view of providing improved conditioning benefits such as slippery feel during the application to wet hair, softness and moisturized feel on dry hair. In at least one embodiment, the fatty alcohol is selected from the group consisting of: cetyl alcohol, stearyl alcohol, behenyl alcohol, and mixtures thereof. In at least one embodiment, the formulation comprises a linear fatty alcohol, wherein the linear fatty alcohol is comprised in a lamellar gel matrix. A lamellar gel matrix is suitable for providing various conditioning benefits such as slippery feel during the application to wet hair and softness and moisturized feel on dry hair. The linear fatty alcohol may comprise from 8 to 24 carbon atoms. In an embodiment, the linear fatty alcohol is selected from the group consisting of: cetyl alcohol, stearyl alcohol, and mixtures thereof. In an embodiment, the weight ratio of total linear fatty alcohol to terminal aminosilicone is from 0.5:1 to 10:1, or from 1:1 to 5:1, or from 2.4:1 to 2.7:1.

In at least one embodiment, the lamellar gel matrix comprises a cationic conditioning surfactant and a high melting point fatty compound. In view of providing the lamellar gel matrix, the cationic conditioning surfactant and the high melting point fatty compound are contained at a level such that the weight ratio of the cationic surfactant to the high melting point fatty compound is in the range of from 1:1 to 1:10, or from 1:1 to 1:6.

In at least one embodiment, the formulation comprises a cationic conditioning surfactant. In at least one embodiment, the formulation comprises from 0.05 % to 3.0 %, or from 0.075 % to 2.0 %, or from 0.1 % to 1.0 %, of cationic conditioning surfactant by total weight of the formulation. In at least one embodiment, the cationic conditioning surfactant is comprised in a lamellar gel matrix. In other words, the formulation comprises a lamellar gel matrix and the lamellar gel matrix comprises the cationic conditioning surfactant. In an embodiment, cationic conditioning surfactant is according to Formula (C): 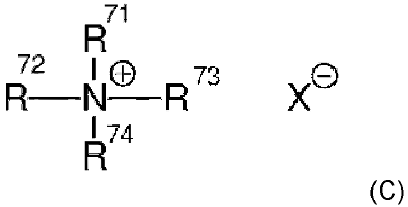
wherein at least one of R⁷¹, R⁷², R⁷³ and R⁷⁴ is selected from an aliphatic group of from 8 to 30 carbon atoms, an aromatic, alkoxy, polyoxyalkylene, alkylamido, hydroxyalkyl, aryl, or an alkylaryl group having up to 22 carbon atoms; the remainder of R⁷¹, R⁷², R⁷³ and R⁷⁴ are independently selected from the group consisting of an aliphatic group of from 1 to 22 carbon atoms, and an aromatic, alkoxy, polyoxyalkylene, alkylamido, hydroxyalkyl, aryl or alkylaryl group having up to 22 carbon atoms;
X is selected from the group consisting of: halogen, acetate, citrate, lactate, glycolate, phosphate, nitrate, sulfonate, sulfate, alkylsulfate, alkyl sulfonate, and combinations thereof.

In at least one embodiment, the cationic conditioning surfactant is selected from the group consisting of behenyl trimethyl ammonium chloride, methyl sulfate or ethyl sulfate, and stearyl trimethyl ammonium chloride, methyl sulfate or ethyl sulfate. It is believed that a longer alkyl group provides improved smoothness and soft feeling on wet and dry hair, compared to cationic surfactants with a shorter alkyl group. It is also believed that such cationic surfactants can provide reduced irritation, compared to those having a shorter alkyl group.

In at least one embodiment, the cationic surfactant is a di-long alkyl quaternized ammonium salt selected from the group consisting of: dialkyl (C14-C18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof.

In at least one embodiment, the cationic surfactant is a tertiary amido amine having an alkyl group of from 12 to 22 carbons. The tertiary amido amine may be selected from the group consisting of stearamidopropyldimethyl-, stearamidopropyldiethyl-, stearamidoethyldiethyl-, stearamidoethyldimethyl-, palmitamidopropyldimethyl-, palmitamidopropyldiethyl-, palmitamidoethyldiethyl-, palmitamidoethyldimethyl-, behenamidopropyldimethyl-, behenamidopropyldiethyl-, behenamidoethyldiethyl-, behenamidoethyldimethyl-, arachidamidopropyldimethyl-, arachidamidopropyldiethyl-, arachidamidoethyldiethyl-, and arachidamidoethyldimethyl-amine, diethylaminoethylstearamide, and mixtures thereof. A tertiary amido amine may be used in combination with an acid. The acid is typically used as a salt-forming anion. In an embodiment, the acid is selected from the group consisting of lactic acid, malic acid, hydrochloric acid, 1-glumatic acid, acetic acid, citric acid, and mixtures thereof.

In at least one embodiment, the cationic surfactant is selected from the group consisting of cetyltrimonium chloride (CTAC), stearyltrimonium chloride (STAC), behentrimonium methosulfate, stearoylamidopropyldimethyl amine (SAPDMA), distearyldimethylammonium chloride, and mixtures thereof.

In at least one embodiment, the formulation comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants. In at least one embodiment, the formulation comprises a total amount of surfactant of from 0.01 wt.-% to 70 wt.-%, from 0.1 wt.-% to 40 %, from 1 wt.-% to 30 %, from 2 wt.-% to 20 wt.-%.

In at least one embodiment, the formulation comprises an anionic surfactant. In at least one embodiment, the anionic surfactant is selected from the group consisting of (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof.

In at least one embodiment, the formulation comprises an acylglycinate surfactant. In at least one embodiment, the acylglycinate surfactant conforms to the formula (Y): 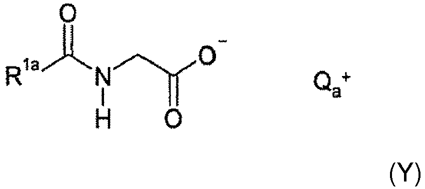 wherein
- R^{1a}: is a linear or branched, saturated alkyl group having 6 to 30, preferably 8 to 22, particularly preferably 8 to 18 carbon atoms or is a linear or branched, mono- or polyunsaturated alkenyl group having 6 to 30, preferably 8 to 22, particularly preferably 12 to 18 carbon atoms, and
- Qₐ⁺: is a cation.

In at least one embodiment, Qₐ⁺ is selected from the group consisting of Li⁺, Na⁺, K⁺, Mg⁺⁺, Ca⁺⁺, Al⁺⁺⁺, NH4⁺, a monoalkylammmonium ion, a dialkylammonium ion, a trialkylammonium ion and a tetraalkylammonium ion, or combinations thereof. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C₁₂ alkyl or C₁₄ alkyl. In at least one embodiment, the acylglycinate surfactant is selected from those conforming to formula (Y), wherein R is C₁₆ alkyl or C₁₈ alkyl.

In at least one embodiment, the formulation comprises a glutamate surfactant corresponding to formula (Z) or a salt thereof: 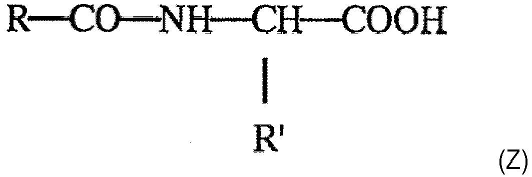 wherein
- R': is HOOC-CH2-CH2- or M⁺⁻OOC-CH2-CH2- wherein M⁺ is a cation;
- R: is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH4⁺, a monoalkylammmonium ion, a dialkylammonium ion, a trialkylammonium ion and a tetraalkylammonium ion, or combinations thereof.

In at least one embodiment, the glutamate surfactant is selected from sodium cocoyl glutamate and potassium cocoyl glutamate. In at least one embodiment, the glutamate surfactant is selected from those conforming to formula (Z), wherein R is C₁₂ alkyl or C₁₄ alkyl. In at least one embodiment, the glutamate surfactant is selected from those conforming to formula (Z), wherein R is C₁₆ alkyl or C₁₈ alkyl.

In at least one embodiment, the formulation comprises from 0.01 wt.-% to 30 wt.-%, preferably from 1 wt.-% to 25 wt.-%, more preferably from 5 wt.-% to 20 wt.-%, particularly preferably from 12 wt.-% to 18 wt.-% anionic surfactant.

In at least one embodiment, the formulation comprises a non-ionic surfactant. In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain having 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C₈- to C₂₂-fatty alcohols, onto C₁₂- to C₂₂-fatty acids or onto alkyl phenols having 8 to 15 carbon atoms in the alkyl group, C₁₂- to C₂₂-fatty acid mono- and diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C₈- to C₂₂-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C₈- to C₂₂-fatty acids and a degree of ethoxylation of from 4 to 20, polyglyceryl fatty acid esters, in particular of one, two or more C₈- to C₂₂-fatty acids and polyglycerol having preferably 2 to 20 glyceryl units, alkyl glucosides, alkyl oligoglucosides and alkyl polyglucosides having C₈ to C₂₂-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (e.g. Pluronics^{®}), fatty acid alkylol amides (fatty acid amide polyethylene glycols), N-alkyl-, N-alkoxypolyhydroxy-fatty acid amides, sucrose esters, sorbitol esters, polyglycol ethers, and mixtures thereof.

In at least one embodiment, the formulation comprises a fatty N-methyl-N-glucamide surfactant, wherein the fatty N-methyl-N-glucamide surfactant conforms to the formula (X): 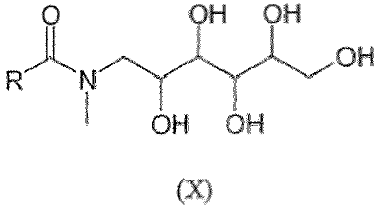 wherein R is selected from saturated or unsaturated hydrocarbon chains having 5 to 23 carbon atoms. Preferably, R in formula (X) is selected from saturated or unsaturated hydrocarbon chains having 7 to 17 carbon atoms. Also preferably, the R-C=O residue in formula (X) is derived from caprylic acid, capric acid, lauric acid, myristic acid, palmitic acid, stearic acid, oleic acid, linoleic acid, linolenic acid, coconut fatty acids, or mixtures thereof. Also preferably, the R-C=O residue in formula (X) is derived from 9-decenoic acid, 9-dodecenoic acid, or mixtures thereof.

Particularly preferred N-methyl-N-acylglucamines of formula (X) are capryloyl/caproyl methyl glucamide, lauroyl/myristoyl methyl glucamide, cocoyl methyl glucamide, oleyl methyl glucamide, or mixtures thereof. Such N-methyl-N-acylglucamines are commercially available from Clariant (GlucoTain^{®} Clear, GlucoTain^{®} Plus, GlucoTain^{®} Flex, GlucoTain^{®} Care, GlucoTain^{®} Sense).

Also particularly preferred N-methyl-N-acylglucamines of formula (X) are N-9-decenoyl-N-methylglucamine, N-9-dodecenoyl-N-methylglucamine, or mixtures thereof.

In at least one embodiment, the formulation comprises from 1 wt.-% to 20 wt.-%, preferably from 2 wt.-% to 10 wt.-%, more preferably from 3 wt.-% to 7 wt.-% non-ionic surfactant.

In at least one embodiment, the amphoteric surfactants are selected from the group consisting of N-(C₁₂-C₁₈)-alkyl-β-aminopropionates and N-(C₁₂-C₁₈)-alkyl-β-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine, (C₁₂-C₁₈)-alkyl-dimethyl-sulfopropylbetaine, amphosurfactants based on imidazoline (e.g. Miranol^{®}, Steinapon^{®}), preferably the sodium salt of 1-(beta-carboxymethyloxyethyl)-1-(carboxymethyl)-2-laurylimidazolinium; amine oxides, e.g. (C₁₂-C₁₈)-alkyldimethylamine oxides, fatty acid amidoalkyldimethylamine oxides, and mixtures thereof.

In at least one embodiment, the formulation comprises a betaine surfactant. Optionally, the betaine surfactant is selected from C₈- to C₁₈-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine, and combinations thereof. Optionally, the betaine surfactant is selected from C₈- to C₁₈-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C₈- to C₁₈-alkyldimethylammonium acetates, the C₈- to C₁₈-alkyldimethylcarbonylmethylammonium salts, and the C₈- to C₁₈-fatty acid alkylamidobetaines, and mixtures thereof. Optionally, the C₈- to C₁₈-fatty acid alkylamidobetaine is selected from coconut fatty acid amidopropylbetaine, N-coconut fatty acid amidoethyl-N-[2-(carboxymethoxy)ethyl]glycerol (CTFA name: Cocoamphocarboxyglycinate), and mixtures thereof.

In at least one embodiment, the formulation comprises from 0.5 wt.-% to 20 wt.-%, preferably from 1 wt.-% to 10 wt.-% amphoteric surfactant.

In at least one embodiment, the formulation comprises a surfactant system. In at least one embodiment, the surfactant system comprises at least one surfactant selected from the group consisting of lauryl sulfate, laureth sulfate, cocoamidopropylbetaine, sodium cocoylglutamate, lauroamphoacetate, and mixtures thereof. In at least one embodiment, the surfactant system comprises sodium laureth sulphate, sodium lauryl sulphate, and optionally cocamidopropyl betaine. In at least one embodiment, the surfactant system comprises sodium laureth sulphate, potassium cocoyl glutamate, and cocamidopropyl betaine.

In at least one embodiment, the formulation further comprises a hairstyling polymer. In at least one embodiment, the hairstyling polymer is selected from the group consisting of: amphoteric hairstyling polymers, zwitterionic hairstyling polymers, anionic hairstyling polymers, non-ionic hairstyling polymers, cationic hairstyling polymers, and mixtures thereof. In at least one embodiment, the formulation comprises from 0.01 % to 20 %, or from 0.01 % to 16 %, or from 0.01 % to 10 %, or from 1 % to 8 %, or from 2 % to 6 % of hairstyling polymer. Suitable hairstyling polymers are disclosed in, e.g., WO2018/002100 (pages 55-63).

In at least one embodiment, the formulation has a viscosity of from 0 cPs to 20,000 cPs. In at least one embodiment, the formulation has a viscosity of from 0.1 cPs to 10,000 cPs, or from 1 cPs to 5,000 cPs, or from 5 cPs to 3,500 cPs.

The viscosity measurement conditions are defined in the definitions section above. Viscosity may be important for anti-drip reasons. Dripping can be inconvenient for the user. Furthermore, more viscous formulations can be useful for measured dispensing. In at least one embodiment, the formulation has a viscosity of from 0 cPs to 1,000 cPs. This viscosity range is advantageous when the formulation is in the form of a facial cleanser in view of the need for distribution on skin and ability to rinse off.

In at least one embodiment, the formulation further comprises a viscosity-modifying substance. The viscosity-modifying substance is preferably a thickening polymer.

In at least one embodiment, the thickening polymer is a polymer based on acrylamidomethylpropanesulfonic acid (AMPS^{®}). These polymers, even at pH values of 7 or less, exhibit good thickening performance. Especially preferably, the thickening polymer is selected from the group consisting of homo- or copolymers of acrylamidomethylpropanesulfonic acid and salts thereof. Among the polymers just mentioned, preference is given to polymers having at least 20 mol-% of units based on acrylamidomethylpropanesulfonic acid and/or salts thereof, and particular preference to polymers having at least 50 mol-% of units based on acrylamidomethylpropanesulfonic acid and/or salts thereof, the mole figures relating in each case to the overall polymer. In the case of the copolymers, in addition to structural units based on acrylamidomethylpropanesulfonic acid and/or salts thereof, preferably one or more structural units based on the following comonomers are present in the copolymers: acrylic acid, methacrylic acid, acrylamide, dimethylacrylamide, vinylpyrrolidone (VP), hydroxyethyl acrylate, hydroxyethyl methacrylate, acrylic or methacrylic esters of ethoxylated alcohols RO-(CH₂CH₂O)ₘH, in which R is an alkyl radical having 12 to 30 carbon atoms and m is a number from 3 to 30, and CH₂=CH-COO-(CH₂CH₂-COO)ₙX, in which n is a number from 0 to 10 and X is a counterion and is preferably H⁺, Na⁺ and/or NH4⁺. The polymers selected from the group consisting of homo- or copolymers of acrylamidomethylpropanesulfonic acid and salts thereof may be crosslinked or non-crosslinked. In the case of crosslinking, they contain structural units based on monomers having 2 or more olefinic double bonds. In the case of crosslinking, preferably from 0.1 to 10 mol-% of such structural units are present in the homo- or copolymers, based on the overall polymer. If one or more structural units based on acrylamidomethylpropanesulfonic acid and/or salts thereof in the homo- or copolymers of acrylamidomethylpropanesulfonic acid and/or salts thereof have one or more counterions other than H⁺, these other counterions are preferably selected from the group consisting of Na⁺ and NH4⁺. Suitable polymers are mentioned in publications including EP-0816403, EP-1069142, EP-1116733 and DE-10 2009 014877 (Clariant), EP-1347736 (L'Oréal) or EP-1496081 (Seppic). Examples include: Aristoflex^{®} AVC (Ammonium Acryloyldimethyltaurate/VP Copolymer), Aristoflex^{®} AVS (Sodium Acryloyldimethyltaurate/VP Crosspolymer), Aristoflex^{®} TAC (Ammonium Acryloyl Dimethyltaurate Carboxyethyl Acrylate Crosspolymer), Hostacerin^{®} AMPS (Ammonium Polyacryloyldimethyl Taurate), Aristoflex^{®} HMB (Ammonium Acryloyldimethyltaurate/Beheneth-25 Methacrylate Crosspolymer), Aristoflex^{®} BLV (Ammonium Acryloyldimethyltaurate/Beheneth-25 Methacrylate Crosspolymer), Aristoflex^{®} HMS (Ammonium Acryloyldimethyltaurate/Steareth-25 Methacrylate Crosspolymer), Aristoflex^{®} SNC (Ammonium Acryloyldimethyltaurate/Steareth-8 Methacrylate Copolymer), Aristoflex^{®} LNC (Ammonium Acryloyldimethyltaurate/Laureth-7 Methacrylate Copolymer) or Sepinov^{®} EMT 10 (Hydroxyethyl Acrylate/Sodium Acryloyldimethyl Taurate Copolymer), Sepigel^{®} 305.

In at least one embodiment, the thickening polymer is selected from the group consisting of: copolymers of at least one first monomer type, which is chosen from acrylic acid and methacrylic acid, and at least one second monomer type, which is chosen from esters of acrylic acid and ethoxylated fatty alcohol; crosslinked polyacrylic acid; crosslinked copolymers of at least one first monomer type, which is chosen from acrylic acid and methacrylic acid, and at least one second monomer type, which is chosen from esters of acrylic acid with C₁₀- to C₃₀-alcohols; copolymers of at least one first monomer type, which is chosen from acrylic acid and methacrylic acid, and at least one second monomer type, which is chosen from esters of itaconic acid and ethoxylated fatty alcohol; copolymers of at least one first monomer type, which is chosen from acrylic acid and methacrylic acid, at least one second monomer type, which is chosen from esters of itaconic acid and ethoxylated C₁₀- to C₃₀-alcohols, and a third monomer type, which is chosen from C₁- to C₄-aminoalkyl acrylates; copolymers of two or more monomers chosen from acrylic acid, methacrylic acid, acrylic esters and methacrylic esters; copolymers of vinylpyrrolidone and ammonium acryloyldimethyltaurate; copolymers of ammonium acryloyldimethyltaurate and monomers chosen from esters of methacrylic acid and ethoxylated fatty alcohols; hydroxyethylcellulose; hydroxypropylcellulose; hydroxypropylguar; glyceryl polyacrylate; glyceryl polymethacrylate; copolymers of at least one C₂-, C₃- or C₄-alkylene and styrene; polyurethanes; hydroxypropyl starch phosphate; polyacrylamide; copolymers of maleic anhydride and methyl vinyl ether crosslinked with decadiene; carob seed flour; guar gum; xanthan; dehydroxanthan; carrageenan; karaya gum; hydrolyzed corn starch; copolymers of polyethylene oxide, fatty alcohols and saturated methylenediphenyl diisocyanate (e.g. PEG-150/stearyl alcohol/SMDI copolymer); and mixtures thereof.

In at least one embodiment, the formulation has a pH value of from 2.0 to 12.0, preferably from 3.0 to 9.0, more preferably from 4.5 to 7.5. By varying the pH value, a formulation can be made available that is suitable for different applications.

In at least one embodiment, the formulation comprises an alkalizing agent or pH adjusting agent. In at least one embodiment, ammonia or caustic soda is suitable, but water-soluble, physiologically tolerable salts of organic or inorganic bases can also be considered. Optionally, the pH adjusting agent is selected from ammonium hydrogen carbonate, ammonia, monoethanolamine, ammonium carbonate. In at least one embodiment, the alkalizing agent or pH adjusting agent is selected from the group consisting of 2-amino-2-methyl-1-propanol, 2-amino-2-methyl-1,3- propanediol, 2-amino-2-ethyl-1,3-propanediol, tris(hydroxymethyl)-aminomethane, 2-amino-1-butanol, tris(2-hydroxypropyl)-amine, 2,2-iminobisethanol, lysine, iminourea (guanidine carbonate), tetrahydro-1,4-oxazine, 2-amino-5-guanidin-valeric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide, magnesium oxide, and mixtures thereof.

To establish an acidic pH value, an acid can be included. In at least one embodiment, the formulation comprises an acid selected from the group consisting of hydrochloric acid, phosphoric acid, acetic acid, formic acid, sulfuric acid, citric acid, and mixtures thereof. Citric acid is most preferred in that it has high consumer acceptance. In at least one embodiment, the acidic pH is adjusted with a buffer such as a phosphate buffer, a TRIS buffer or a citric buffer. The buffers may be used alone or in combination with an acid.

In at least one embodiment, the formulation is in liquid form. In an alternative embodiment, the formulation is in solid form. Optionally, the formulation is in powdered or granulated form. This is advantageous in that it is not needed to ship liquid, which is typically heavy, over long distances, which has economic and environmental benefits. A solid form can be achieved by spray drying the formulation or by using a rotary evaporator. The formulation can be converted into liquid form after it has been shipped, e.g. by adding water.

In at least one embodiment, the formulation is a household cleaning formulation.

In at least one embodiment, the formulation is a hand dishwashing formulation. In at least one embodiment, the hand dishwashing formulation comprises an anionic surfactant. In at least one embodiment, the hand dishwashing formulation comprises from 5 wt.-% to 25 wt.-% anionic surfactant. In at least one embodiment, the hand dishwashing formulation comprises a surfactant system comprising at least one anionic surfactant and a further surfactant selected from non-ionic surfactants, amphoteric surfactants, zwitterionic surfactants, and combinations thereof.

Preferably, the hand dishwashing formulation comprises cocoamidopropylbetaine or an amine oxide. Preferably, the amine oxide is lauryl amine oxide, cocoyl amine oxide, or a combination thereof. In at least one embodiment, the pH value of the hand dishwashing formulation is between pH 5.0 and pH 10, preferably between pH 5.5 and pH 9.0. In the case of the hand dishwashing formulation comprising an amine oxide, the hand dishwashing formulation preferably has a pH of between pH 7.5 and pH 9.5, most preferably between pH 8.0 and pH 9.0.

In at least one embodiment, the formulation is a hard surface cleaner. In at least one embodiment, the hard surface cleaner comprises an anionic surfactant. In at least one embodiment, the hard surface cleaner comprises from 1 wt.-% to 10 wt.-% anionic surfactant. In at least one embodiment, the hard surface cleaner comprises a nonionic surfactant. In at least one embodiment, the hard surface cleaner comprises from 1 wt.-% to 10 wt.-% nonionic surfactant. In at least one embodiment, the hard surface cleaner comprises a surfactant system comprising at least one anionic surfactant and a further surfactant selected from non-ionic surfactants, amphoteric surfactants, zwitterionic surfactants, and combinations thereof. Preferably, the hard surface cleaner comprises linear alkylbenzene sulfonate and fatty alcohol ethoxylate. In at least one embodiment, the pH value of the hard surface cleaner is between pH 5.0 and pH 11, preferably between pH 6.0 and pH 9.0.

In at least one embodiment, the formulation is a liquid laundry detergent formulation comprising one or more surfactants. Preferably, the one or more surfactants of the liquid laundry detergent formulation are selected from the group consisting of anionic, nonionic, cationic and zwitterionic surfactants, and more preferably from the group consisting of anionic, nonionic and zwitterionic surfactants.

### Anionic Surfactants

In at least one embodiment, the formulation comprises an anionic surfactant. Anionic surfactants are particularly useful in cleaning formulations such as household cleaning formulations. Preferred anionic surfactants are alkyl sulfonates and alkyl ether sulfates. Preferred alkyl sulfonates are alkylbenzene sulfonates, particularly linear alkylbenzene sulfonates (LAS) having an alkyl chain length of C₈-C₁₅, preferably C₁₂-C₁₄. Possible counterions for concentrated alkaline liquids are ammonium ions, e.g. those generated by the neutralization of alkylbenzene sulfonic acid with one or more ethanolamines, for example monoethanolamine (MEA) and triethanolamine (TEA), or alternatively, alkali metals, e.g. those arising from the neutralization of alkylbenzene sulfonic acid with alkali hydroxides.

Preferred alkyl ether sulfates (AES) are alkyl polyethoxylate sulfate anionic surfactants.

### Nonionic Surfactants

In at least one embodiment, the formulation comprises a nonionic surfactant. Nonionic surfactants include primary and secondary alcohol ethoxylates, especially C₈-C₂₀ aliphatic alcohols ethoxylated with an average of from 1 to 20 moles of ethylene oxide per mole of alcohol, and more especially the C₁₀-C₁₅ primary and secondary aliphatic alcohols ethoxylated with an average of from 1 to 10 moles of ethylene oxide per mole of alcohol. Non-ethoxylated nonionic surfactants include alkyl polyglycosides, glycerol monoethers and polyhydroxy amides (glucamides). Mixtures of nonionic surfactants may also be used.

When included therein, the household cleaning formulation, particularly the liquid laundry detergent formulation, preferably comprises from 0.2 wt.-% to 40 wt.-%, more preferably from 1 wt.-% to 20 wt.-% nonionic surfactant, such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, N-acyl N-alkyl derivatives of glucosamine (glucamides), or combinations thereof. Nonionic surfactants that may be used include the primary and secondary alcohol ethoxylates, especially the C₈-C₂₀ aliphatic alcohols ethoxylated with an average of from 1 to 35 moles of ethylene oxide per mole of alcohol, and more especially the C₁₀-C₁₅ primary and secondary aliphatic alcohols ethoxylated with an average of from 1 to 10 moles of ethylene oxide per mole of alcohol.

### Zwitterionic Surfactants

In at least one embodiment, the formulation comprises a zwitterionic surfactant. The liquid laundry detergent formulation may comprise a zwitterionic surfactant, e.g. amine oxide or betaine, preferably in an amount of up to 10 wt.-% based on the total weight of the liquid laundry detergent formulation. Betaines may be alkyldimethyl betaines or alkylamido betaines, wherein the alkyl groups have C₁₂-C₁₈ chains.

### Additional Surfactants

In at least one embodiment, the liquid laundry detergent formulation comprises a surfactant selected from the group consisting of anionic surfactants, nonionic surfactants, and mixtures thereof; preferably the surfactant is selected from the group consisting of linear alkyl benzene sulfonates, alkyl ether sulfates, nonionic surfactants, amine oxides and betaines; and more preferably selected from the group consisting of linear alkyl benzene sulfonates, alkyl ether sulfates and nonionic surfactants. Other surfactants than the preferred LAS, AES, and nonionic surfactants may also be added. Although less preferred, alkyl sulfate surfactants may be used, especially the non-ethoxylated C₁₂-C₁₅ primary and secondary alkyl sulfates. Soap may also be used. Levels of soap are preferably lower than 10 wt.-%.

Preferably, the one or more surfactants in the liquid laundry detergent formulations are present in an amount of at least 5 wt.-%, preferably from 5 wt.-% to 65 wt.-%, more preferably from 6 wt.-% to 60 wt.-% and particularly preferably from 7 wt.-% to 55 wt.-%, in each case based on the total weight of the liquid laundry detergent formulation.

### Further Optional Ingredients

The household cleaning formulations may comprise one or more optional ingredients, e.g. they may comprise conventional ingredients commonly used in detergent compositions, especially laundry detergent compositions. Examples of optional ingredients include, but are not limited to builders, bleaching agents, bleach active compounds, bleach activators, bleach catalysts, photobleaches, dye transfer inhibitors, color protection agents, anti-redeposition agents, dispersing agents, fabric softening and antistatic agents, fluorescent whitening agents, enzymes, enzyme stabilizing agents, foam regulators, defoamers, malodor reducers, preservatives, disinfecting agents, hydrotropes, fibre lubricants, anti-shrinkage agents, buffers, fragrances, processing aids, colorants, dyes, pigments, anti-corrosion agents, fillers, stabilizers or other conventional ingredients for washing or laundry detergent compositions.

### Polymer

For detergency boosting, it may be advantageous to use a polymer in the household cleaning formulations, particularly in the liquid laundry detergent formulations. This polymer is preferably a polyalkoxylated polyethyleneimine (EPEI). Polyethylene imines are materials composed of ethylene imine units -CH₂CH₂NH- and, where branched, the hydrogen on the nitrogen is replaced by another chain of ethylene imine units. These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, or the like. Specific methods for preparing these polyamine backbones are disclosed in US 2,182,306, US 3,033,746, US 2,208,095, US 2,806,839, and US 2,553,696.

The household cleaning formulations, particularly the liquid laundry detergent formulations, may comprise other polymeric materials, for example: dye transfer inhibition polymers, anti-redeposition polymers or cotton soil release polymers, especially those based on modified cellulosic materials. Especially, when EPEI is not present, the formulation may further comprise a polymer of polyethylene glycol and vinyl acetate, for example the lightly grafted copolymers described in WO 2007/138054. Such amphiphilic graft polymers based on water soluble polyalkylene oxides as graft base and side chains formed by polymerization of a vinyl ester component have the ability to enable reduction of surfactant levels whilst maintaining high levels of oily soil removal.

### Hydrotropes

In at least one embodiment, the formulation comprises a hydrotrope. Herein "hydrotrope" is a solvent that is neither water nor conventional surfactant, and that aids the solubilisation of surfactants and other components, especially any polymer and/or sequestrant, in the liquid, to render it isotropic. Hydrotropes are particularly useful in household cleaning formulations. Among suitable hydrotropes the following are noteworthy: monopropylene glycol (MPG), glycerol, sodium cumene sulfonate, ethanol, other glycols, e.g. dipropylene glycol, diethers and urea. MPG and glycerol are preferred hydrotropes.

### Enzymes

In at least one embodiment, the formulation, particularly the liquid laundry detergent formulation, comprises an enzyme. In at least one embodiment, the enyzme is selected from the group consisting of protease, mannanase, pectate lyase, cutinase, esterase, lipase, amylase, cellulase, and combinations thereof. Less preferred additional enzymes may be selected from peroxidase and oxidase. The enzymes are preferably present with corresponding enzyme stabilizers. The total enzyme content in the formulation is preferably from 0 wt. % to 5 wt.-%, more preferably from 0.5 wt.-% to 5 wt.-%, even more preferably from 1 wt.-% to 4 wt.-%, by total weight of the formulation.

### Sequestrants

Sequestrants are preferably included in the formulation, particularly in the household cleaning formulations. Preferred sequestrants include organic phosphonates, alkanehydroxy phosphonates and carboxylates, for example available under the DEQUEST trade mark from Thermphos. The preferred sequestrant level is less than 10 wt.-% and preferably less than 5 wt.-% by total weight of the formulation. A particularly preferred sequestrant is HEDP (1-hydroxyethylidene-1,1-diphosphonic acid), for example sold as Dequest 2010. Also suitable is Dequest^{®} 2066 (diethylenetriamine penta(methylene-phosphonic acid) or Heptasodium DTPMP).

### Buffers

In at least one embodiment, the formulation, particularly the liquid laundry detergent formulation, comprises a buffer. In addition to agents optionally included for the generation of anionic surfactants, e.g. from LAS or fatty acids, the presence of buffer is preferred for pH control. Possible buffers are one or more ethanolamines, e.g. monoethanolamine (MEA) or triethanolamine (TEA). They are preferably used in formulation at levels of from 1.0 wt.-% to 15 wt.-%. Other suitable amino alcohol buffer materials may be selected from the group consisting of compounds having a molecular weight above 61 g/mol, which includes MEA. Suitable materials also include, in addition to the already mentioned materials: monoisopropanolamine, diisopropanolamine, triisopropanolamine, monoamino hexanol, 2-[(2-methoxyethyl)methylamino]-ethanol, propanolamine, N-methylethanolamine, diethanolamine, monobutanolamine, isobutanolamine, monopentanolamine, 1-amino-3-(2-methoxyethoxy)-2-propanol, 2-methyl-4-(methylamino)-2-butanol, or mixtures thereof. Potential alternatives to amino ethanol buffers are alkali hydroxides such as sodium hydroxide or potassium hydroxide.

In at least one embodiment, the formulation is a lubricant, a paint or a coating. The paints may, for example, be decorative paints. The coatings may, for example, be architectural coatings. The coatings may, for example, be water-based coatings.

The present invention also relates to the use of a compound of Formula (I) of the present invention or an antimicrobial blend of the present invention in a cosmetic formulation, a household cleaning formulation, a lubricant, a paint or a coating.

The present invention also relates to the use of a compound of Formula (I) of the present invention or an antimicrobial blend of the present invention as an antimicrobial agent.

In at least one embodiment, the antimicrobial agent is an antibacterial agent or an antifungal agent. In at least one embodiment, the antimicrobial agent is an antibacterial agent. In at least one embodiment, the antimicrobial agent is an antifungal agent.

The present invention also relates to a method of reducing the effects of microorganisms in a formulation, wherein the method comprises adding a compound of Formula (I) of the present invention or an antimicrobial blend of the present invention to the formulation.

In at least one embodiment, the microorganisms are bacteria or fungi. In at least one embodiment, the microorganisms are bacteria. In at least one embodiment, the microorganisms are fungi. Fungi may, for example, be yeast or mold.

In this document, including in all embodiments of all aspects of the present invention, the following definitions apply unless specifically stated otherwise. All percentages are by weight (w/w) of the total composition. All ratios are weight ratios. "wt.-%" means percentage by weight. References to 'parts' e.g. a mixture of 1 part X and 3 parts Y, is a ratio by weight. "QS" or "QSP" means sufficient quantity for 100 % or for 100 g. +/- indicates the standard deviation. All ranges are inclusive and combinable. The number of significant digits conveys neither a limitation on the indicated amounts nor on the accuracy of the measurements. All measurements are understood to be made at 23°C and at ambient conditions, where "ambient conditions" means at 1 atmosphere (atm) of pressure and at 50 % relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR^{®} International. Herein "min" means "minute" or "minutes". Herein "mol" means mole. Herein "g" following a number means "gram" or "grams". Herein, "comprising" means that other steps and other ingredients can be in addition. "Comprising" encompasses the terms "consisting of" and "consisting essentially of". The compositions, formulations, methods, uses, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition.

"Molecular weight" or "M.Wt." or "MW" and grammatical equivalents mean the number average molecular weight.

"Viscosity" is measured at 25°C using a HAAKE Rotation Viscometer VT 550 with cooling/heating vessel and sensor systems according to DIN 53019 at a shear rate of 12.9 s⁻¹.

"Water-soluble" refers to any material that is sufficiently soluble in water to form a clear solution to the naked eye at a concentration of 0.1 % by weight of the material in water at 25°C. The term "water-insoluble" refers to any material that is not "water-soluble".

"Dry" or "substantially dry" means comprising less than 5 %, less than 3 %, less than 2 %, less than 1 %, or about 0 % of any compound or composition being in liquid form when measured at 25°C at ambient conditions. Such compounds or compositions being in liquid form include water, oils, organic solvents and other wetting agents. "Anhydrous" means that the composition comprises less than 5 %, less than 3 %, less than 2 %, less than 1 %, or about 0 % water by total weight of the composition.

"Substantially free from" or "substantially free of" means less than 1 %, or less than 0.8 %, or less than 0.5 %, or less than 0.3 %, or about 0 %, by total weight of the composition or formulation.

"Derivatives" includes but is not limited to, amide, ether, ester, amino, carboxyl, acetyl, acid, salt and/or alcohol derivatives of a given compound. In at least one embodiment, "derivatives thereof" means the amide, ether, ester, amino, carboxyl, acetyl, acid, salt and alcohol derivatives.

The examples which follow are intended to illustrate the subject matter of the invention without restricting it thereto.

### EXAMPLES

### Materials

"Phenoxyethanol (25% bio-based)" as used in the following Examples 1 to 9 refers to phenoxyethanol having 25 wt-% bio-based carbon content.

"Phenoxyethanol (75% bio-based)" as used in the following Examples 1 to 9 refers to phenoxyethanol having 75 wt-% bio-based carbon content.

"Phenoxyethanol (100% bio-based)" as used in the following Examples 1 to 9 refers to phenoxyethanol having 100 wt-% bio-based carbon content.

### Example 1

Example blends of the present invention (amounts are given in wt.-%)

| | Example | | | | | | | | | |
|---|---|---|---|---|---|---|---|---|---|---|
| | 1a | 1b | 1c | 1d | 1e | 1f | 19 | 1h | 1i | 1j |
| Phenoxyethanol (25% bio-based) | 85 | - | - | - | - | - | - | 60 | - | - |
| Phenoxyethanol (75% bio-based) | - | 82 | - | - | 70 | - | - | - | 80 | - |
| Phenoxyethanol (100% bio-based) | - | - | 70 | 90 | - | 90 | 90 | - | - | 30 |
| Ethylhexylglycerin | 15 | - | - | - | - | 10 | - | - | - | - |
| Caprylyl Glyceryl Ether | - | 14 | - | - | - | - | 10 | - | - | - |
| Sorbitan Caprylate | - | - | 30 | - | - | - | - | 40 | - | - |
| Caprylyl glycol | - | - | - | 10 | - | - | - | - | 20 | - |
| Capryloyl/Caproyl Anhydro Methyl Glucamide | - | - | - | - | 20 | - | - | - | - | 50 |
| Water | - | 4 | - | - | 10 | - | - | - | - | 20 |
| Total [%] | 100 | 100 | 100 | 100 | 100 | 100 | 100 | 100 | 100 | 100 |

The blends are prepared by mixing its ingredients at room temperature.

### Example 2

Example blends of the present invention (amounts are given in wt.-%)

| | Example | | | | | | | | | |
|---|---|---|---|---|---|---|---|---|---|---|
| | 2a | 2b | 2c | 2d | 2e | 2f | 2g | 2h | 2i | 2j |
| Phenoxyethanol (25% bio-based) | - | 90 | - | - | - | - | 79 | - | 63 | - |
| Phenoxyethanol (75% bio-based) | 60 | - | - | 80 | - | 70 | - | - | - | - |
| Phenoxyethanol (100% bio-based) | - | - | 81 | - | 85 | - | - | 70 | - | 70 |
| Piroctone Olamine | - | 4 | - | - | - | - | 11 | - | - | - |
| Benzoic acid | - | - | 7 | 20 | - | 15 | - | - | - | - |
| Dehydroacetic acid | - | - | 12 | - | 15 | - | - | - | - | - |
| Caprylyl glycol | 15 | - | - | - | - | - | - | - | - | 10 |
| Hydroxyacetophenone | 25 | - | - | - | - | - | - | - | 27 | - |
| Caprylhydroxamic acid | - | - | - | - | - | - | - | 7,5 | - | 10 |
| Water | - | 6 | - | - | - | - | - | - | 10 | - |
| 1,2-Propanediol | - | - | - | - | - | 15 | 10 | 22.5 | - | 10 |
| Total [%] | 100 | 100 | 100 | 100 | 100 | 100 | 100 | 100 | 100 | 100 |

The blends are prepared by mixing its ingredients at room temperature.

### Example 3

Example blends of the present invention (amounts are given in wt.-%)

| | Example | | | | | | | | | |
|---|---|---|---|---|---|---|---|---|---|---|
| | 3a | 3b | 3c | 3d | 3e | 3f | 3g | 3h | 3i | 3j |
| Phenoxyethanol (25% bio-based) | 73 | - | 97 | - | 10 | 50 | - | 70 | - | - |
| Phenoxyethanol (75% bio-based) | - | 70 | - | - | - | - | 45 | - | 60 | - |
| Phenoxyethanol (100% bio-based) | - | - | - | 90 | - | - | - | - | - | 50 |
| Benzylalcohol | - | - | - | - | - | 50 | 55 | - | - | - |
| Hydroxyethoxyphenyl Butanone | - | - | - | - | - | - | - | 30 | - | - |
| Methylpropandiol | - | - | - | - | - | - | - | - | 40 | - |
| Methylheptandiol | - | - | - | - | - | - | - | - | - | 50 |
| Methylparaben | 15 | 10 | - | - | - | - | - | - | - | - |
| Ethylparaben | 4 | 10 | - | - | - | - | - | - | - | - |
| Propylparaben | 8 | 10 | - | - | - | - | - | - | - | - |
| Chlorphenesin | - | - | - | 10 | 90 | - | - | - | - | - |
| Methylisothiazolinone | - | - | 1 | - | - | - | - | - | - | - |
| Water | - | - | 2 | - | - | - | - | - | - | - |
| Total [%] | 100 | 100 | 100 | 100 | 100 | 100 | 100 | 100 | 100 | 100 |

The blends are prepared by mixing its ingredients at room temperature.

### Example 4

Example formulations of the present invention (amounts are given in wt.-%)

| | Example | | | | |
|---|---|---|---|---|---|
| | 4a | 4b | 4c | 4d | 4e |
| Formulation | Liquid soap | Body wash | Wet wipe lotion | Shampoo | Shampoo |
| Phenoxyethanol (100% bio-based) | 0.7 | 0.9 | 1 | 0.9 | 0.8 |
| Ethylhexylglycerin | - | - | - | 0.1 | - |
| Benzoic acid | 0.5 | 0.1 | - | - | - |
| Sorbitan Caprylate | 1 | - | 0.5 | - | - |
| Capryloyl/Caproyl Anhydro Methyl Glucamide | - | - | - | - | 1 |
| Caprylyl glycol | 10 | - | - | - | 9 |
| Cocamidopropylbetaine | 2 | 4.5 | - | - | 2 |
| Cocamide MEA | - | - | - | - | 1 |
| Sodium Cocoyl Glutamate | - | 2 | 1 | 2 | - |
| Sodium Cocoyl Glycinate | - | 2 | 1 | 2 | - |
| Sodium Cocoyl Isethionate | - | 2 | | - | - |
| Cocoyl Methyl Glucamide | - | 3 | 2 | - | - |
| EGDS | - | 0.5 | - | - | 0.7 |
| PEG-7 Glyceryl Cocoate | - | - | 1 | - | - |
| Cocoyl Glucoside | - | - | - | 5 | - |
| Lauryl Glucoside | - | - | - | 5 | - |
| Acrylates Copolymer | - | - | - | 2 | - |
| Glycerin | - | - | - | 1 | 2 |
| Panthenol | - | - | - | 0.2 | 0.2 |
| Dimethicone | - | - | - | - | - |
| Polyquaternium 7 (PQ-7) | - | - | - | - | 0.6 |
| Guar Hydroxypropyltrimonium Chloride | - | - | - | - | 0.3 |
| Hydrolyzed Protein | - | - | - | - | 0.1 |
| Perfume | 0.2 | 0.2 | 0.1 | - | 0.8 |
| NaCl | 1.5 | 1.0 | 0.5 | 0.3 | 1.5 |
| Water | QSP | QSP | QSP | QSP | QSP |
| Total | 100 % | 100 % | 100 % | 100 % | 100 % |

### Example 5

Example formulations of the present invention (amounts are given in wt.-%)

| | Example | | | | |
|---|---|---|---|---|---|
| | 5a | 5b | 5c | 5d | 5e |
| Formulation | Hand dishwashing formulation | | | | |
| Phenoxyethanol (25% bio-based) | - | - | 1.5 | - | 1 |
| Phenoxyethanol (100% bio-based) | 1 | 1 | - | 2 | - |
| Benzoic acid | 2.5 | 1.5 | - | - | - |
| Methylisothiazolinone | - | - | 0.0015 | - | - |
| Benzisothiazolinone | - | - | 0.0050 | - | - |
| Potassium sorbate | - | - | - | 0.6 | 0.5 |
| Sorbitan Caprylate | 1 | 1 | - | 1 | 1 |
| Sodium Lauryl Ether Sulfate (SLES) | 18 | 18 | 19 | 8 | - |
| Cocamidopropylbetaine | 8 | - | - | 4 | - |
| Sodium Alkane Sulfonate | - | - | - | 24 | - |
| Lauryldimethylamine oxide | - | - | 6 | - | 5 |
| Ethanol | 7 | 4 | - | - | - |
| Coco-Glucamide | - | 8 | - | - | - |
| Perfume | 0.2 | 0.2 | 0.1 | 0.1 | 0.8 |
| Cumene Sulfonate | - | - | 1.5 | - | - |
| Alpha-Olefine Sulfonate (Na salt) | - | - | - | - | 15 |
| Water & Auxiliaries | QSP | QSP | QSP | QSP | QSP |
| Total | 100 % | 100 % | 100 % | 100 % | 100 % |

### Example 6

Example formulations of the present invention (amounts are given in wt.-%)

| | Example | | | |
|---|---|---|---|---|
| | 6a | 6b | 6c | 6d |
| Formulation | Body lotion | Intensive hand and body lotion | Anti-ageing night cream | Baby cream |
| Phenoxyethanol (25% bio-based) | - | - | 1 | - |
| Phenoxyethanol (100% bio-based) | 1 | 1 | - | 0.7 |
| Caprylyl glycol | - | - | 0.5 | - |
| Ethylhexylglycerin | - | 0.2 | - | - |
| Caprylyl Glyceryl Ether | 0.5 | - | - | - |
| Sorbitan caprylate | - | - | - | 0.3 |
| Ethylhexyl Stearate | 7 | - | - | - |
| Decyl Oleate | 5 | - | - | - |
| Plantasens^{®} Natural Emulsifier HE 20 (Clariant), which is Cetearyl Glucoside (and) Sorbitan Olivate | 3 | - | - | - |
| Dimethicone | 2 | - | - | - |
| Glycerin | 3 | 3 | 7 | - |
| Xanthan Gum | 0.2 | - | - | - |
| Aristoflex^{®} AVC from Clariant (Ammonium Acryloyldimethyltaurate/ VP Copolymer) | 0.5 | 1 | - | - |
| Fragrance | 0.3 | - | 0.3 | 0.3 |
| Polyglyceryl-2-Sesquiisostearate | - | 0.5 | - | 2 |
| Trilaureth-4 Phosphate | - | 2 | - | - |
| Mineral Oil (Paraffinum Liquidum) | - | 5 | - | - |
| Plantasens^{®} Refined Organic Babassu Butter from Clariant (Orbignya Oleifera Seed Oil) | - | 2 | - | - |
| Squalane | - | 2 | 12 | - |
| Macadamia Ternifolia Seed Oil (and) Crambe Abyssinica Seed Oil (and) Orbignya Oleifera Seed Oil | - | 2 | - | - |
| Silcare^{®} Silicone SEA from Clariant (Trideceth-9 PG-Amodimethicone and Trideceth-12) | - | 0.5 | - | - |
| Cetearyl Alcohol | - | 2 | - | - |
| Isopropyl Palmitate | - | 4 | - | - |
| Dow Corning^{®} 345 (Cyclopentasiloxane and Cyclohexasiloxane) | - | - | 10 | - |
| Paraffin Oil, low viscosity | - | - | 4 | 10 |
| Petrolatum | - | - | 4 | 15 |
| Cetyl Alcohol | - | - | 3 | - |
| Cutina^{®} GMS (Glyceryl Stearate) | - | - | 2.5 | - |
| PEG-40 Stearate | - | - | 3 | - |
| Cera Alba Wax | - | - | 2 | - |
| Mangifera Indica (Mango) Seed Butter | - | - | 2 | - |
| Abyssinian Oil (and) Phytosterols (and) Olea Europea (Olive) Oil Unsaponifiables | - | - | 0.5 | - |
| Sorbitan Tristearate | - | - | 0.3 | - |
| Ubiquinone | - | - | 0.05 | - |
| Aristoflex^{®} Velvet from Clariant (Polyacrylate Crosspolymer-11) | - | - | 0.3 | - |
| Hostacerin^{®} SFO from Clariant (Sunflower Seed Oil Sorbitol Esters) | - | - | - | 3.5 |
| Paracera^{®} M (Cera Microcristallina) | - | - | - | 2 |
| Beeswax | - | - | - | 1 |
| Magnesium Stearate | - | - | - | 1 |
| Talc | - | - | - | 10 |
| Zinc Oxide | - | - | - | 10 |
| Allantoin (Clariant) | - | - | - | 0.3 |
| Extrapon Hamamelis | - | - | - | 2 |
| D-Panthenol | - | - | - | 2 |
| Water | QSP | QSP | QSP | QSP |
| Total | 100 % | 100 % | 100 % | 100 % |

### Example 7

Example formulations of the present invention (amounts are given in wt.-%)

| | Example | | | | |
|---|---|---|---|---|---|
| | 7a | 7b | 7c | 7d | 7e |
| Formulation | Hard surface cleaner | | | | |
| Phenoxyethanol (25% bio-based) | - | 1.5 | - | - | 1 |
| Phenoxyethanol (75% bio-based) | - | - | 1 | - | - |
| Phenoxyethanol (100% bio-based) | 1 | - | - | 2 | - |
| Benzoic acid | 2.5 | 1.5 | - | - | - |
| Methylisothiazolinone | - | - | 0.0015 | - | - |
| Benzisothiazolinone | - | - | 0.0050 | - | - |
| Potassium sorbate | - | - | - | 0.6 | 0.5 |
| Sodium Lauryl Ether Sulfate (SLES) | 2 | 2 | 2 | - | - |
| Lauryldimethylamine Oxide | 2 | - | - | - | 2 |
| Alkylbenzenesulfonate | - | - | - | 2 | 2 |
| Laureth-7 | - | - | 2 | 2 | |
| Capric/Caprylic-Glucamide | - | 2 | - | - | - |
| Perfume | 0.2 | 0.2 | 0.1 | 0.1 | 0.8 |
| Water & Auxiliaries | QSP | QSP | QSP | QSP | QSP |
| Total | 100 % | 100 % | 100 % | 100 % | 100 % |

### Example 8

Example formulations of the present invention (amounts are given in wt.-%)

| | Example | | | | |
|---|---|---|---|---|---|
| | 8a | 8b | 8c | 8d | 8e |
| Formulation | Liquid laundry detergent formulation | | | | |
| Phenoxyethanol (25% bio-based) | - | 1.5 | - | - | 1 |
| Phenoxyethanol (75% bio-based) | - | - | 1 | - | - |
| Phenoxyethanol (100% bio-based) | 1 | - | - | 2 | - |
| Benzoic acid | 2.5 | 1.5 | - | - | - |
| Methylisothiazolinone | - | - | 0.0015 | - | - |
| Benzisothiazolinone | - | - | 0.0050 | - | - |
| Potassium sorbate | - | - | - | 0.6 | 0.5 |
| Sodium Lauryl Ether Sulfate (SLES) | 10 | 15 | 5 | 15 | 2 |
| Alkylbenzenesulfonate | 10 | 5 | 15 | 0 | 15 |
| Laureth-7 | 10 | 10 | 10 | 10 | 8 |
| C₁₂/C₁₄ Fatty Acid Soap | 3 | 3 | 3 | 5 | 5 |
| Perfume | 0.2 | 0.2 | 0.1 | 0.1 | 0.8 |
| Water & Auxiliaries | QSP | QSP | QSP | QSP | QSP |
| Total | 100 % | 100 % | 100 % | 100 % | 100 % |

### Example 9

Example formulations of the present invention (amounts are given in wt.-%)

| | Example | | | | | |
|---|---|---|---|---|---|---|
| | 9a | 9b | 9c | 9d | 9e | 9f |
| Formulation | Soap formulation | Soap bar | Body wash | Shampoo | Wash cream | Body wash |
| Phenoxyethanol (100% bio-based) | 0.6 | 0.7 | 0.8 | 0.5 | 0.5 | 0.5 |
| Benzoic acid | 0.2 | 0.2 | - | 0.3 | - | 0.3 |
| Sorbitan caprylate | - | - | - | - | - | 1 |
| Potassium sorbate | 0.2 | - | - | - | - | - |
| Capryloyl/Caproyl Anhydro Methyl Glucamide | - | - | 1 | - | - | - |
| Benzyl alcohol | - | - | - | 0.5 | - | - |
| Caprylyl Glyceryl Ether | - | - | - | - | 0.5 | - |
| Sodium Lauryl Sulfate | - | - | 10 | - | - | - |
| Ammonium Lauryl Sulfate | - | - | - | 12 | - | - |
| Sodium Lauryl Ether Sulfate (SLES) | 15 | - | 2 | - | - | - |
| Cocamidopropylbetaine | 7 | - | 2 | 3 | - | 2 |
| Cocamide MEA | - | - | 1 | - | - | 2 |
| Lauric Acid | 0.5 | 2 | - | - | - | 10 |
| Myristic Acid | 1.5 | 2 | - | - | - | 10 |
| Stearic Acid | 0.5 | 2 | - | - | 10 | - |
| Palmitic Acid | - | 2 | - | - | - | 10 |
| Potassium Tallowate | - | 2 | - | - | - | - |
| Sodium Palm Kernelate | - | 20 | - | - | - | - |
| Sodium Cocoate | - | 60 | - | - | - | - |
| Lauryl Glucoside | - | - | - | 4 | - | - |
| Sodium Cocoyl Isethionate | - | - | - | - | 14 | - |
| Glycerin | - | 5 | - | - | - | 5 |
| Cetearyl Alcohol | 1.5 | - | - | - | 2 | - |
| Dimethicone | - | - | - | - | 0.1 | - |
| Polyquaternium 7 (PQ-7) | - | - | - | 0.2 | - | - |
| Guar Hydroxypropyltrimonium Chloride | - | - | 0.3 | - | - | - |
| Hydrolyzed Protein | - | - | - | 0.1 | - | - |
| Perfume | 1 | 0.2 | 0.1 | - | 0.8 | 0.7 |
| NaCl | 1.5 | - | 0.5 | 0.3 | - | 2.5 |
| Water | QSP | QSP | QSP | QSP | QSP | QSP |
| Total | 100% | 100% | 100% | 100% | 100% | 100% |

## Claims

1. A compound of Formula (I) 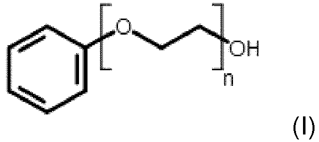
wherein n is an integer from 1 to 10; and
wherein the compound of Formula (I) has at least 10 wt-%, preferably at least 25 wt-%, more preferably at least 50 wt-%, even more preferably at least 75 wt-%, particularly preferably 100 wt-%, bio-based carbon content, relative to the total mass of carbon in the compound.

2. The compound according to claim 1, wherein n is 1, 2 or 3, preferably n is 1 or 2, particularly preferably n is 1.

3. The compound according to claim 1 or 2, wherein the compound of Formula (I) is phenoxyethanol.

4. The compound according to any of claims 1 to 3, wherein the compound of Formula (I) is derived from ethylene oxide, wherein the ethylene oxide has at least 10 wt-%, preferably at least 25 wt-%, more preferably at least 50 wt-%, even more preferably at least 75 wt-%, particularly preferably 100 wt-%, bio-based carbon content, relative to the total mass of carbon in the ethylene oxide.

5. The compound according to any of claims 1 to 4, wherein the compound of Formula (I) is derived from phenol, wherein the phenol has at least 10 wt-%, preferably at least 25 wt-%, more preferably at least 50 wt-%, even more preferably at least 75 wt-%, particularly preferably 100 wt-%, bio-based carbon content, relative to the total mass of carbon in the phenol.

6. A process for preparing a compound of Formula (I) 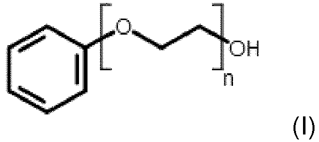
wherein n is an integer from 1 to 10; and
wherein the compound of Formula (I) has at least 10 wt-%, preferably at least 25 wt-%, more preferably at least 50 wt-%, even more preferably at least 75 wt-%, particularly preferably 100 wt-%, bio-based carbon content, relative to the total mass of carbon in the compound;
wherein the process comprises reacting phenol with ethylene oxide,
wherein
the phenol has at least 10 wt-%, preferably at least 25 wt-%, more preferably at least 50 wt-%, even more preferably at least 75 wt-%,
particularly preferably 100 wt-%, bio-based carbon content, relative to the total mass of carbon in the phenol,
and/or
the ethylene oxide has at least 10 wt-%, preferably at least 25 wt-%, more preferably at least 50 wt-%, even more preferably at least 75 wt-%,
particularly preferably 100 wt-%, bio-based carbon content, relative to the total mass of carbon in the ethylene oxide.

7. The process according to claim 6, wherein the phenol is reacted with the ethylene oxide in the presence of a base.

8. An antimicrobial blend comprising
(A) a compound of Formula (I) as defined in any of claims 1 to 5, as component A; and
(B) an antimicrobial agent or a preservative booster, as component B.

9. The antimicrobial blend according to claim 8, wherein the weight ratio of component A to component B is from 1:20 to 20:1.

10. The antimicrobial blend according to claim 8 or 9, wherein the antimicrobial agent (component B) is selected from the group consisting of aromatic alcohols, organic acids and salts thereof, hydroxamic acids and salts thereof, hydroxypyridones, alkyl diols, halogenated compounds, isothiazolinones, and mixtures thereof.

11. The antimicrobial blend according to any of claims 8 to 10, wherein the preservative booster (component B) is selected from the group consisting of
compounds of Formula (II) 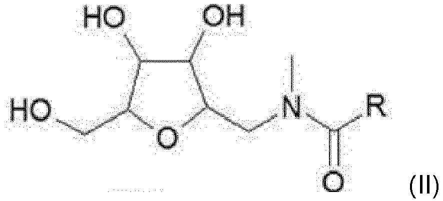
wherein R is selected from saturated hydrocarbon chains having 5 to 23 carbon atoms, unsaturated hydrocarbon chains having 5 to 23 carbon atoms, and mixtures thereof,
sorbitan esters, isosorbide esters, glyceryl ethers, and glyceryl esters.

12. A formulation comprising
(a) from 0.01 to 10 wt-%, preferably from 0.1 to 2 wt-%, of a compound of Formula (I) as defined in any of claims 1 to 5 or an antimicrobial blend as defined in any of claims 8 to 11; and
(b) from 90 to 99.99 wt-%, preferably from 98 to 99.9 wt-%, of one or more further components.

13. Use of a compound of Formula (I) as defined in any of claims 1 to 5 or an antimicrobial blend as defined in any of claims 8 to 11 in a cosmetic formulation, a household cleaning formulation, a lubricant, a paint or a coating.

14. Use of a compound of Formula (I) as defined in any of claims 1 to 5 or an antimicrobial blend as defined in any of claims 8 to 11 as an antimicrobial agent.
